(19) **Europäisches Patentamt / European Patent Office / Office européen des brevets**

(11) **EP 4 129 169 B1**

(12) **EUROPEAN PATENT SPECIFICATION**

(45) Date of publication and mention
of the grant of the patent:
**06.05.2026 Bulletin 2026/19**

(21) Application number: **20926577.6**

(22) Date of filing: **23.03.2020**

(51) International Patent Classification (IPC):
*G16H 20/40* (2018.01)   *G16H 40/63* (2018.01)
*A61B 5/0205* (2006.01)   *A61B 5/08* (2006.01)
*A61B 5/087* (2006.01)   *A61B 5/00* (2006.01)
*A61M 16/00* (2006.01)   *A61B 5/01* (2006.01)
*A61B 5/021* (2006.01)   *A61B 5/091* (2006.01)
*A61B 5/145* (2006.01)   *A61B 5/318* (2021.01)
*A61M 16/01* (2006.01)   *A61M 16/20* (2006.01)
*A61M 16/22* (2006.01)

(52) Cooperative Patent Classification (CPC):
**A61B 5/0816; A61B 5/0205; A61B 5/087;
A61B 5/4836; A61M 16/0051; A61M 16/024;
G16H 20/40; G16H 40/63;** A61B 5/01; A61B 5/021;
A61B 5/091; A61B 5/14542; A61B 5/318;
A61B 2505/01; A61B 2505/03;   (Cont.)

(86) International application number:
**PCT/CN2020/080701**

(87) International publication number:
**WO 2021/189198 (30.09.2021 Gazette 2021/39)**

(54) **METHOD AND APPARATUS FOR MONITORING VENTILATION OF PATIENT**

VERFAHREN UND VORRICHTUNG ZUR ÜBERWACHUNG DER BEATMUNG EINES PATIENTEN

PROCÉDÉ ET APPAREIL DE SURVEILLANCE DE LA VENTILATION D'UN PATIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.02.2023 Bulletin 2023/06**

(73) Proprietor: **Shenzhen Mindray Bio-Medical
Electronics Co., Ltd.
Shenzhen, Guangdong 518057 (CN)**

(72) Inventors:
• **ZOU, Xinru
Shenzhen, Guangdong 518057 (CN)**
• **LIU, Jinglei
Shenzhen, Guangdong 518057 (CN)**
• **ZHOU, Xiaoyong
Shenzhen, Guangdong 518057 (CN)**

(74) Representative: **KIPA AB
Drottninggatan 11
252 21 Helsingborg (SE)**

(56) References cited:
CN-A- 101 380 233    CN-A- 102 415 883
CN-A- 105 899 249    CN-A- 109 303 960
US-A1- 2003 111 078    US-A1- 2007 028 921
US-A1- 2008 281 219    US-A1- 2009 062 674

(52) Cooperative Patent Classification (CPC): (Cont.)
A61B 2505/05; A61M 16/01; A61M 16/0891;
A61M 16/202; A61M 16/208; A61M 16/22;
A61M 2016/0027; A61M 2016/0036;
A61M 2016/0039; A61M 2016/0042;

A61M 2205/3331; A61M 2205/3344;
A61M 2205/3561; A61M 2205/3569;
A61M 2205/3592; A61M 2205/505; A61M 2205/583;
A61M 2230/04; A61M 2230/205; A61M 2230/30;
A61M 2230/40; A61M 2230/50

**EP 4 129 169 B1**

**Description**

TECHNICAL FIELD

The disclosure relates to a method and apparatus for monitoring ventilation of a patient.

BACKGROUND

Human respiration refers to periodic rhythmic inhalation and exhalation of gas, absorption of oxygen and discharge of carbon dioxide, which are for gas exchange. When some patients are unable to breathe spontaneously, mechanical ventilation process can be configured to help them finish respiration. For example, if the patient does not breathe spontaneously, external devices, such as a ventilator can usually be configured to provide ventilatory support to the patient. It can be seen that mechanical ventilation process is a ventilation method that uses a mechanical device to replace, control or change the spontaneous ventilation action of the patient. However, mechanical ventilation process is also easy to cause lung injury (ventilator induced lung injury, VILI) to the patients. Typically, during the mechanical ventilation process, improper setting of related mechanical device, such as ventilator, causes lung injury to the patients. Therefore, how to control, regulate and preset parameters of related mechanical devices to avoid lung injury caused by mechanical ventilation process, and even in turn to improve the lung related parameters of the patients, is a problem that technicians have been studying.

US2007/028921A1 teaches a method of controlling pulmonary ventilation for a ventilator utilize a trainable neural network to determine the desired level settings of the ventilator setting controls, and employ such desired level settings of the ventilator setting controls and the ventilation data output signal to control the ventilator.

US2009/062674A teaches a spirometry-based method for calculating work of breathing (WOB) based on an airway gas flow and an airway pressure. However, neither of these prior arts mentions monitoring ventilation of a patient.

SUMMARY

This disclosure mainly provides a method and apparatus for monitoring ventilation of a patient.

According to a first aspect, an embodiment provides a method for monitoring ventilation of a patient, including:

acquiring a pressure of the patient during a mechanical ventilation process, wherein the pressure reflects a pressure which acts on different sites of a respiratory system of the patient during the mechanical ventilation process;
acquiring a gas flow rate of the patient during the mechanical ventilation process;
calculating, according to the acquired pressure and the acquired gas flow rate, energy which acts on the respiratory system of the patient during the mechanical ventilation process;
acquiring a ventilation parameter; and
determining a degree of contribution of the ventilation parameter to the energy which acts on the respiratory system of the patient during the mechanical ventilation process.

In one embodiment, the ventilation parameter includes a ventilation control parameter and/or a respiratory system related parameter; wherein the ventilation control parameter comprises one or more of tidal volume, gas flow rate, drive pressure, positive respiratory end pressure, respiratory rate and respiratory ratio, and the respiratory system related parameter comprises one or more of respiratory system compliance and respiratory system resistance.

In an embodiment, the method for monitoring ventilation of a patient further includes displaying the degree of contribution of each ventilation parameter.

In an embodiment, the degree of contribution of each ventilation parameter is displayed in one or more manners of word, number, character, table, diagram or icon.

In an embodiment, the method for monitoring ventilation of a patient further includes guiding the mechanical ventilation process according to the degree of contribution.

In an embodiment, guiding the mechanical ventilation process according to the degree of contribution, includes: indicating to decrease the ventilation parameter according to a ranking of the degree of contribution or controlling to decrease the ventilation parameter according to a ranking of the degree of contribution, when determining that the energy which acts on the respiratory system of the patient during the mechanical ventilation process, exceeds a first threshold.

In an embodiment, guiding the mechanical ventilation process according to the degree of contribution, includes: indicating to increase the ventilation parameter according to a ranking of the degree of contribution or controlling to increase the ventilation parameter according to a ranking of the degree of contribution, when determining that the energy which acts on the respiratory system of the patient during the mechanical ventilation process, is lower than a second threshold.

In an embodiment, guiding the mechanical ventilation process according to the degree of contribution, includes: estimating and outputting the energy which acts on the respiratory system of the patient, during the mechanical ventilation process which is set according to a setting command of a ventilation control parameter.

In an embodiment, guiding the mechanical ventilation process according to the degree of contribution, further includes:

determining whether to send an alarm according to the estimated energy which acts on the respiratory system of the patient, during the mechanical ventilation process.

In an embodiment, guiding the mechanical ventilation process according to the degree of contribution, includes: determining whether to send an alarm according to the degree of contribution of each respiratory system related parameter.

According to the second aspect, an embodiment provides an apparatus for monitoring ventilation of a patient is provided, including:

a pressure sensor, which is configured to acquire a pressure of a patient during a mechanical ventilation process, wherein the pressure reflects a pressure which acts on different sites of a respiratory system of the patient during the mechanical ventilation process;

a flow sensor, which is configured to acquire a gas flow rate of the patient during the mechanical ventilation process; and

a processor, which is configured to acquire the pressure of the patient during the mechanical ventilation process and the gas flow rate of the patient during the mechanical ventilation process, and to calculate, according to the acquired pressure and the acquired gas flow rate, energy which acts on the respiratory system of the patient during the mechanical ventilation process; wherein the processor is further configured to acquire a ventilation parameter, and determine a degree of contribution of the ventilation parameter to the energy which acts on the respiratory system of the patient during the mechanical ventilation process.

In one embodiment, the ventilation parameter includes a ventilation control parameter and/or a respiratory system related parameter; wherein the ventilation control parameter includes one or more of tidal volume, a gas flow rate, drive pressure, positive respiratory end pressure, respiratory rate and respiratory ratio, and the respiratory system related parameter includes one or more of respiratory system compliance and respiratory system resistance.

In an embodiment, the apparatus for monitoring ventilation of a patient further includes a display, which is configured to display the degree of contribution of each ventilation parameter.

In an embodiment, the processor is further configured to guide the mechanical ventilation process according to the degree of contribution.

In an embodiment, in order to guide the mechanical ventilation process according to the degree of contribution, the processor is further configured to:

indicate to preferentially decrease the ventilation parameter with a largest degree of contribution among ventilation control parameters, or control to preferentially decrease the ventilation parameter with a largest degree of contribution among ventilation control parameters, when determining that the energy which acts on the respiratory system of the patient during the mechanical ventilation process, exceeds a first threshold; and/or

indicate to preferentially increase the ventilation parameter with a smallest degree of contribution among ventilation control parameters, or control to preferentially increase the ventilation parameter with a smallest degree of contribution among ventilation control parameters, when determining that the energy which acts on the respiratory system of the patient during the mechanical ventilation process, is lower than a second threshold.

In an embodiment, in order to guide the mechanical ventilation process according to the degree of contribution, the processor is further configured to:

estimate and output the energy which acts on the respiratory system of the patient, during the mechanical ventilation process which is set according to a setting command of a ventilation control parameter.

In an embodiment, in order to guide the mechanical ventilation process according to the degree of contribution, the processor is further configured to:

determine whether to send an alarm according to the estimated energy which acts on the respiratory system of the patient, during the mechanical ventilation process.

In an embodiment, in order to guide the mechanical ventilation process according to the degree of contribution, the processor is further configured to:

determine whether to send an alarm according to the degree of contribution of each respiratory system related parameter.

In an embodiment, the apparatus for monitoring ventilation of a patient is a patient monitor, a patient monitoring module or a medical ventilation apparatus.

According to a third aspect, an embodiment provides a computer-readable storage medium, which includes a program, wherein when the program is executed by a processor, the above method according to any embodiment of this disclosure is implemented.

BRIEF DESCRIPTION OF THE DRAWINGS

FIG. 1 is a structural diagram of an apparatus for monitoring ventilation of a patient according to an embodiment.
FIG. 2 is a structural diagram of an apparatus for monitoring ventilation of a patient according to another embodiment.
FIG. 3 is a structural diagram of an apparatus for monitoring ventilation of a patient according to a further embodiment.
FIG. 4 is a structural diagram of an apparatus for monitoring ventilation of a patient according to another further embodiment.
FIG. 5 is a schematic diagram of a static pressure-volume curve of a respiratory system according to an embodiment.
FIG. 6 is a schematic diagram of a static pressure-volume curve of a respiratory system according to another embodiment.
FIG. 7 (a) and FIG. 7 (b) are two schematic diagrams showing degree of contribution.
FIG. 8 is a flow diagram of a method for monitoring ventilation of a patient according to an embodiment.
FIG. 9 is a flow diagram of a method for monitoring ventilation of a patient according to another embodiment.
FIG. 10 is a flow diagram of a method for monitoring ventilation of a patient according to a further embodiment.
FIG. 11 is a flow diagram of a method for monitoring ventilation of a patient according to another further embodiment.

DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the related operations according to the description in this disclosure and the general technical knowledge in the art.

In addition, the features, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are operable to distinguish different objects, rather than to describe a specific order. In addition, the terms "connection", and "linkage" mentioned in this application include direct and indirect connection (linkage), unless otherwise specified.

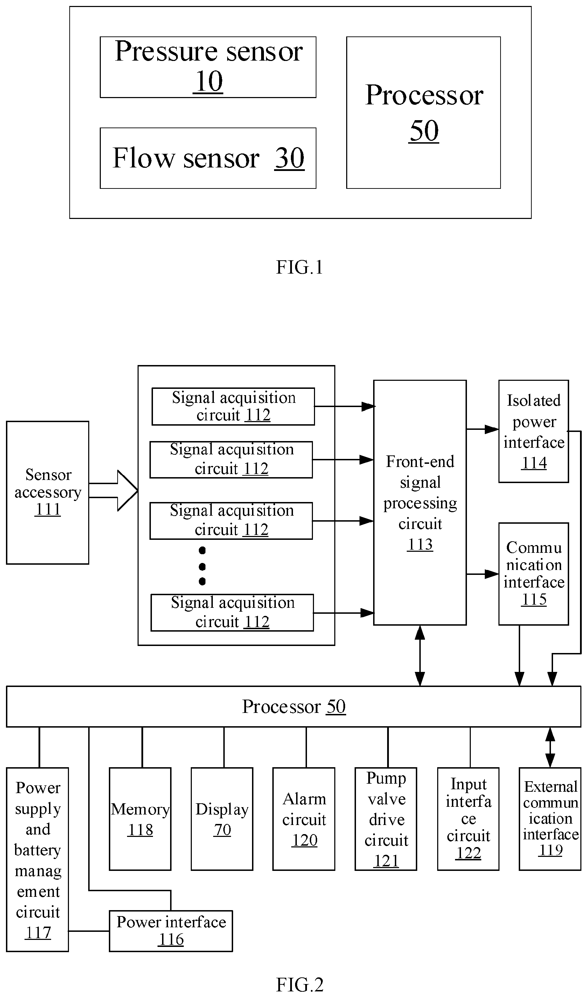

Some embodiments of the disclosure disclose an apparatus for monitoring ventilation of a patient. Referring to FIG. 1, in some embodiments, the apparatus for monitoring ventilation of a patient may include a pressure sensor 10, a flow sensor 30 and a processor 50. The apparatus for monitoring ventilation of a patient in this disclosure may be applied to a variety of occasions. For example, the apparatus for monitoring ventilation of a patient in this disclosure can be a patient monitor or a patient monitoring module in some embodiments, and can be a medical ventilation device in some embodiments, such as a ventilator and an anesthetic machine, which are described below respectively.

In some embodiments, the apparatus for monitoring ventilation of a patient may be a patient monitor.

Referring FIG.2, in some embodiments, the apparatus for monitoring ventilation of a patient has an independent housing, whose panel is provided with a sensor interface zone which is integrated therein multiple sensor interfaces for connecting with various external physiological parameter sensor accessories 111, or in some embodiment for connecting with the above pressure sensor 10 and flow sensor 30. The housing panel also includes one or more of a small IxD display zone, display 70, an input interface circuit 122 and an alarm circuit 120 (such as, a LED alarm zone) and the likes. The apparatus for monitoring ventilation of a patient includes an external communication interface 119 for communicating with a main unit of a medical device (such as a patient monitor, ventilator, anesthetic machine, etc.) and a power interface 16 for taking power from the main unit of the medical device. The apparatus for monitoring ventilation of a patient also supports a build-out parameter module, can form a plug-in monitor main unit by means of inserting the parameter module, can be used as part of the monitor, or can be connected to the main unit via a cable, with the build-out parameter module being used as an external accessory of the monitor. The internal circuit of the apparatus for monitoring ventilation of a patient is disposed inside the housing. The internal circuit includes a signal acquisition circuit 112 corresponding to one or more physiological parameters, and a front-end signal processing circuit 113. The signal acquisition circuit 112 may be selected from an electrocardiogram circuit, a body temperature circuit, a blood oxygen saturation circuit, a non-invasive blood pressure circuit, an invasive blood pressure circuit, etc. These signal acquisition circuits 112 are electrically connected with corresponding sensor interfaces for electrically connecting to sensor accessories 111 corresponding to different physiological parameters. An output terminal of the signal acquisition circuit 112 is coupled to the front-end signal processing circuit 113 whose communication terminal is further coupled to the processor 50. The processor 50 is electrically connected with the external communication interface 119 and the power interface 116. Various general circuits known in the prior art can be configured to realize the signal acquisition circuit 112 and sensor accessories 111 corresponding to different physiological parameters. The front-end signal processing circuit 113 can be configured to complete the sampling and analog-to-digital conversion of output signals of the signal acquisition circuit 112, and output control signals to control the physiological parameter measurement process. The physiological parameters include but are not limited to parameters of electrocardiogram, body temperature, blood oxygen saturation, non-invasive blood pressure, and invasive blood pressure. The front-end signal processing circuit113 can be realized by a single-chip microcomputer or other semiconductor devices, such as mixed signal single-chip microcomputer of LPC2136 which is produced by PHLIPS Corp., or ADuC7021 which is produced by ADI Corp, or by ASIC or FPGA. The front-end signal processing circuit 113 can be powered by an isolated power supply. After a simple processing and packaging, the sampled data can be sent to the processor 50 through an isolated communication interface. For example, the front-end signal processing circuit 113 can be coupled to the processor 50 through an isolated power interface 114 and a communication interface 115. Supplying electrical power to the front-end signal processing circuit 113 through the isolated power supply has a function of isolating the patient from the power supply device through isolating the DC/DC power supply via a transformer. In such a way, 1, the application part is floating through the isolation transformer, such that the leakage current passing through the patient is small enough, and 2, bad influences on boards and devices of intermediate circuits, such as main control board (guaranteed by creepage distance and electrical clearance), due to voltage or energy generated during a defibrillation or electric knife application, can be prevented. Of course, the front-end signal processing circuit 113 can be coupled to the processor 50 directly through cables. The processor 50 completes the calculation of physiological parameters and sends calculation results and waveforms of the physiological parameter to the main unit (such as, a main unit with a display, PC, a central station, etc.) through the external communication interface 119. The processor 50 can be directly connected with the external communication interface 119 through cables for communication and be directly connected with the external communication and power interface 116 through cables for taking power. The apparatus for monitoring ventilation of a patient may also include a power supply and battery management circuit 117, which takes power from the main unit through the power interface 116 and supplies it to the processor 50 after processing, such as rectification and filtering. The power supply and battery management circuit 117 can also monitor, manage and protect the power obtained from the main unit through the power interface 116. The external communication interface 119 may be one or a combination of local area network interfaces composed of Ethernet, Token Ring, Token Bus, and optical fiber distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB. The external communication interface 119 may also be one or a combination of the wireless data transmission interface and the wired data transmission interface. The main unit may be any computer device such as a main unit of the monitor, a computer, and the likes, and a monitor can be formed by means of installing with matching software on the main unit. The main unit may also be a communication device, such as a mobile phone, and the apparatus for monitoring ventilation of a patient sends data to a mobile phone that supports Bluetooth communication via a Bluetooth interface to realize remote data transmission. After the processor 50 completes the calculation of physiological parameters, it can also determine whether the physiological parameters are abnormal. If yes, it can send an alarm through the alarm circuit 120. The memory 118 may store intermediate and final data of the monitor, as well as program instructions or code for execution by the processor 50 or the like. If the monitor has the function of blood pressure measurement, it may also include a pump valve drive circuit 121, which is configured to implement inflation or deflation under the control of the processor 50.

The above are some descriptions of apparatus for monitoring ventilation of a patient as a patient monitor.

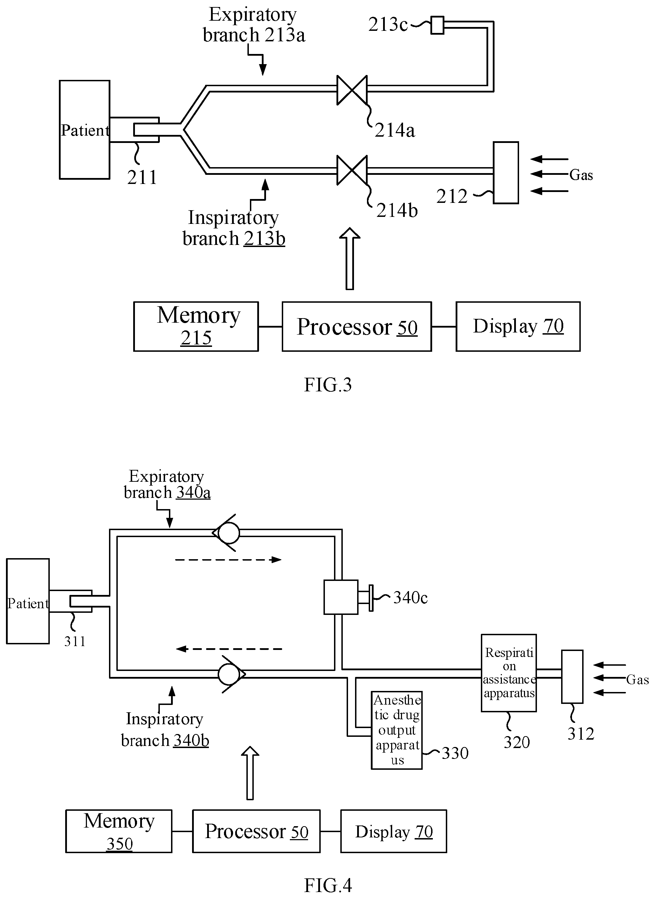

In some embodiments, the apparatus for monitoring ventilation of a patient can also be a ventilator, which is an artificial mechanical ventilation apparatus to assist or control the spontaneous respiration movement of the patient, so as to achieve the function of gas exchange in the lung, decrease the consumption of the human body, and facilitate the recovery of respiratory function. Referring to FIG. 3, in some embodiments, the apparatus for monitoring ventilation of a patient may also include a respiratory interface 211, a gas source interface 212, a respiratory line, a respiration assist apparatus and a display 70.

The respiratory line selectively connects the gas source interface 212 with the respiratory system of the patient. In some embodiments, the respiratory line includes an inspiratory branch 213b and an expiratory branch 213a. The expiratory branch 213a is connected between the respiratory interface 211 and an exhaust port 213c, and is configured to export the exhaled gas of the patient to the exhaust port 213c. The exhaust port 213c can be connected to the external environment, and can also be connected into a dedicated gas recovery apparatus. The gas source interface 212 is configured to connect with the gas source (not shown), which is configured to provide gas. The gas can usually be oxygen, air and so on. In some embodiments, the gas source can be a compressed gas cylinder or a central gas supply source, which supplies gas to the ventilator through the gas source interface 212. The types of gas include oxygen $O_2$, air, etc. The gas source interface 212 can include conventional components, such as pressure gauge, pressure regulator, flowmeter, pressure-reduction valve and air-oxygen proportional control protection device, which are respectively configured to control the flow of various gases (such as oxygen and air). The inspiratory branch 213b is connected between the respiratory interface 211 and the gas source interface 212 to provide oxygen or air for the patient. For example, the gas which is inputted from the gas source interface 212, enters the inspiratory branch 213b, and then enters the lungs of the patient through the respiratory interface 211. The respiratory interface 211 is configured to connect the patient to the respiratory line. In addition to guiding the gas transmitted by the inspiratory branch 213b to the patient, the exhaled gas of the patient can also be guided to the exhaust port 213c through the expiratory branch 213a. Depending on the situation, the respiratory interface 211 may be a nasal cannula or a mask for wearing on the mouth and nose. The respiration assistance apparatus is connected with the gas source interface 212 and the respiratory line, and controls the gas provided by the external gas source to be transmitted to the patient through the respiratory line. In some embodiments, the respiration assistance apparatus may include an expiratory controller 214a and an inspiratory controller 214b. The expiratory controller 214a is arranged at the expiratory branch 213a to switch on or off the expiratory branch 213a according to the control command, or to control the flow rate or pressure of the exhaled gas of the patient. When specifically implemented, the exhalation controller 214a may include one or more elements that can control flow or pressure, such as an exhalation valve, a check valve, a flow controller, a PEEP (positive expiratory end pressure) valve, etc. The inspiratory controller 214b is arranged at the inspiratory branch 213b to switch on or off the inspiratory branch 213b according to the control command, or to control the flow rate or pressure of the output gas. When specifically implemented, the inspiratory controller 214b may include one or more elements that can control flow or pressure, such as an exhalation valve, a check valve, a flow controller, etc.

The memory 215 may be configured to store data or program, such as data sampled by each sensor, data generated by the processor through calculation, or image frames generated by the processor. The image frames may be 2D or 3D images. Optionally, the memory 215 may store a graphical user interface, one or more default image display settings, and programming instructions for the processor. The memory 215 may be a tangible and non-transient computer-readable medium, such as flash memory, RAM, ROM, EEPROM, and the like.

In some embodiments, the processor 50 is configured to execute instructions or programs, control various control valves in the respiration assist apparatus, the gas source interface 212 and/or the respiratory line, or process the received data, generate the required calculation or determination results, or generate visual data or graphics, and output the visual data or graphics to the display 70 for displaying.

The above are some descriptions of apparatus for monitoring ventilation of a patient as a ventilator. It should be noted that FIG. 3 above is only an example of a ventilator, which is not configured to limit the ventilator to such a structure.

In some embodiments, the apparatus for monitoring ventilation of a patient can also be an anesthetic machine, which is mainly configured to provide anesthetic gas, send the anesthetic gas to the respiratory system of the patient through a ventilator, and control the amount of anesthetic gas inhaled. Referring to FIG. 4, the apparatus for monitoring ventilation of a patient of some embodiments may also include a respiratory interface 311, a gas source interface 312, a respiration assistance apparatus 320, an anesthetic drug output apparatus 330, a respiratory line, a memory 350 and a display 70.

The gas source interface 312 is configured to connect with the gas source (not shown), which is configured to provide gas. The gas can usually be oxygen, nitrous oxide (laughing gas), air and so on. In some embodiments, the gas source can be a compressed gas cylinder or a central gas supply source, which supplies gas to the anesthetic machine through the gas source interface 312. The types of gas include oxygen $O_2$, nitrous oxide $N_2O$, air, etc. The gas source interface 312 can include conventional components, such as pressure gauge, pressure regulator, flowmeter, pressure-reduction valve and $N_2O$-$O_2$ proportional control protection device, which are respectively configured to control the flow volume of various gases (such as oxygen, laughing gas and air). The gas, which is inputted from the gas source interface 312, enters the respiratory line and forms a mixed gas with the original gas in the respiratory line.

The respiration assistance apparatus 320 is configured to provide power to the patient for the non-spontaneous respiration, maintain the airway unblocked. In some embodiments, the respiration assistance apparatus 320 is connected with the gas source interface 312 and the respiratory line to control the delivery of the gas provided by the external gas source to the patient through the respiratory line. In some specific embodiments, the respiration assistance apparatus 320 mixes the fresh gas which is inputted from the gas source interface 312 with the gas which is exhaled by the patient in the respiratory line and the anesthetic drug which is outputted from the anesthetic drug output apparatus 330, and outputs the mixture to the respiratory interface 311 through the inspiratory branch 340b to drive the patient to inhale, and receives the exhaled gas from the patient through the expiratory branch 340a. In a specific embodiment, the respiration assistance apparatus 320 usually includes a machine-controlled ventilation module, and the gas duct of the machine-controlled ventilation module is connected with the respiratory line. During the anesthesia maintenance stage of the surgery or the state when the patient does not resume spontaneous respiration, the machine-controlled ventilation module is configured to provide the patient with respiratory power. In some embodiments, the respiration assistance apparatus 320 also includes a manual ventilation module, and the gas duct of the manual ventilation module is connected with the respiratory line. During the induction stage before intubation, the manual ventilation module is usually configured to assist the patient in respiration. When the respiration assistance apparatus 320 includes both machine-controlled ventilation module and manual ventilation module, the machine-controlled ventilation mode and the manual ventilation mode can be switched through a machine-controlled or manual switch (such as a three-way valve), so as to connect the machine-controlled ventilation module or manual ventilation module with the respiratory line for controlling the respiration of the patient. Those skilled in the art should understand that the anesthetic machine can only include a machine-controlled ventilation module or a manual ventilation module according to specific requirements.

The anesthetic drug output apparatus 330 is configured to provide anesthetic drugs. Generally, the anesthetic drugs are mixed into fresh air which is guided by the gas source interface 312 in the form of gas and delivered to the respiratory line together. In a specific embodiment, the anesthetic drug output apparatus 330 is realized by using an anesthetic drug volatilization tank. Anesthetic drugs are usually liquid and stored in the anesthetic drug volatilization tank. Optionally, the anesthetic drug volatilization tank can include a heating device to heat the anesthetic drug to volatilize and generate anesthetic steam. The anesthetic drug output apparatus 330 is connected with the pipeline of the gas source interface 312. The anesthetic steam is mixed with the fresh air which is guided from the gas source interface 312 and then transmitted to the respiratory line together with the fresh air.

In some embodiments, the respiratory line includes an inspiratory branch 340b, an expiratory branch 340a and a soda-lime tank 340c, wherein the inspiratory branch 340b and the expiratory branch 340a are connected to form a closed loop. The soda-lime tank 340c is arranged at the expiratory branch 340a. The mixed gas of fresh air which is guided from the gas source interface 312, is inputted from an inlet of the inspiratory branch 340b and supplied to the patient 20 through the respiratory interface 311 which arranged at an outlet of the inspiratory branch 340b. The respiratory interface 311 may be a mask, a nasal intubation, or an endotracheal intubation. In a preferred embodiment, the inspiratory branch 340b is provided with a check valve, which opens in the inspiratory phase and closes in the expiratory phase. The expiratory branch 340a is also provided with a check valve, which is closed in the inspiratory phase and opened in the expiratory phase. The inlet of the expiratory branch 340a is connected with the respiratory interface 311. When the patient exhales, the exhaled gas enters the soda-lime tank 340c through the expiratory branch 340a. The carbon dioxide in the exhaled gas is filtered by the substances in the soda-lime tank 340c, and the filtered gas is recycled into the inspiratory branch 340b.

The memory 350 may be configured to store data or program, such as data sampled by each sensor, data generated by the processor through calculation, or image frames generated by the processor. The image frames may be 2D or 3D images. Optionally, the memory 350 may store a graphical user interface, one or more default image display settings, and programming instructions for the processor. The memory 350 may be a tangible and non-transient computer-readable medium, such as flash memory, RAM, ROM, EEPROM, and the like.

In some embodiments, the processor 50 is configured to execute instructions or programs, control various control valves in the respiration assistance apparatus 320, the gas source interface 310 and/or the respiratory line, or process the received data, generate the required calculation or determination results, or generate visual data or graphics, and output the visual data or graphics to the display 70 for displaying.

The above are some descriptions of apparatus for monitoring ventilation of a patient as an anesthetic machine. It should be noted that FIG. 4 above is only an example of anesthetic machine, which is not configured to limit the anesthetic machine to such a structure.

Lung injury VILI is a combination of various types of injury, which is caused by excessive dynamic strain and energy load. In some embodiments, lung injury can be evaluated more accurately, truly and in real time by calculating the energy which acts on the respiratory system of the patient during the mechanical ventilation process. The following describes how the apparatus for monitoring ventilation of a patient in this disclosure calculate the energy which acts on the respiratory system of the patient during the mechanical ventilation process.

The flow sensor 30 can be configured to acquire a gas flow rate of the patient during the mechanical ventilation process. In some embodiments, the gas flow rate of the patient during the mechanical ventilation process can refer to inspiratory flow rate of the patient. In some embodiments, the flow sensor 30 may be a flow sensor arranged at the patient end, such as a flow sensor arranged at the patient interface, and the gas flow rate is the gas flow rate which is acquired by the flow sensor during the inspiratory phase. In some embodiments, there are multiple flow sensors 30, which includes an inspiratory flow sensor and an expiratory flow sensor which are arranged at the mechanical ventilation end. For example, for a ventilator, the flow sensor 30 may be an inspiratory flow sensor arranged at the inspiratory branch 213b and an expiratory flow sensor arranged at the expiratory branch 213a. For an anesthetic machine, the flow sensor 30 may be an inspiratory flow sensor arranged at the inspiratory branch 340b and an expiratory flow sensor arranged at the expiratory branch 340a. The gas flow rate is the difference between the flow rates acquired by the inspiratory flow sensor and the expiratory flow sensor during the inspiratory period. In some embodiments, the flow sensor can also be a Y-piece flow sensor, which directly measures the inflow and outflow flow rates at the patient end as the gas flow rate. Of course, the energy which acts on the respiratory system of the patient during the mechanical ventilation process can be calculated by the gas flow rate during the whole respiratory period, including the gas flow rate during inspiratory phase and expiratory phase.

In some embodiments, the pressure sensor 10 may be one or more pressure sensors 10. The pressure sensor 10 is configured to acquire the pressure of the patient during the mechanical ventilation process, which pressure reflects the pressure which acts on different points of the respiratory system of the patient during the mechanical ventilation process, such as one or more of airway pressure, intrathoracic pressure, carinal pressure, intrapulmonary pressure, esophageal pressure and intragastric pressure.

In some embodiments, the pressure sensor 10 may be a pressure sensor, such as a catheter-type pressure sensor or an optical fiber-type pressure sensor. By inserting the pressure sensor to the corresponding site of the respiratory system of the patient, the pressure at the corresponding site can be acquired. For example, if the pressure sensor is inserted into the airway of the patient, the airway pressure can be acquired. If the pressure sensor is inserted into the esophagus, the esophageal pressure can be acquired. If the pressure sensor is inserted into the stomach, the intragastric pressure can be acquired. If the pressure sensor is inserted into the carina inside the trachea, the carinal pressure can be acquired. If the pressure sensor is inserted into the stomach, the intragastric pressure can be acquired. If the pressure sensor is inserted into the thoracic cavity through a wound incision, the intrathoracic pressure can be acquired. In some embodiments, the pressures at some points in the respiratory system can also be used to replace or calculate the pressure at other points, which is illustrated by several examples below.

In some embodiments, the carinal pressure may be used to replace the intrapulmonary pressure. In some embodiments, the esophageal pressure may be used to replace the intrathoracic pressure. In some embodiments, the intragastric pressure may be used to replace the intraabdominal pressure.

In some embodiments, the processor 50 may calculate intrapulmonary pressure according to the airway pressure. For example, in some embodiments, the processor 50 calculates the intrapulmonary pressure according to the airway pressure, the respiratory system resistance, and the gas flow rate described above. In a specific example, it can be calculated by the following formula:

$$Plung(t)=Paw(t)-Raw*Flow(t).$$

Wherein Plung(t) represents a function of intrapulmonary pressure which changes with time t, or represents a real-time intrapulmonary pressure. *Paw*(*t*) represents a function of airway pressure which changes with time t, or represents a real-time airway pressure. *Flow*(*t*) represents a function of the gas flow rate of the patient during the mechanical ventilation process which changes with time t, or represents a real-time gas flow rate of the patient during the mechanical ventilation process. Raw represents a respiratory system resistance.

In some embodiments, the processor 50 may calculate transpulmonary pressure by subtracting either the esophageal pressure or the intrathoracic pressure from either the intrapulmonary pressure or the airway pressure. For example, the transpulmonary pressure is obtained by subtracting the esophageal pressure from the airway pressure. In some embodiments, the processor 50 may also correct the transpulmonary pressure, which is described in detail below.

In some embodiments, the processor 50 also corrects the transpulmonary pressure by the airway pressure value and the esophageal pressure value in the states where the positive expiratory end pressure is zero and non-zero. Specifically, the processor 50 acquires the airway pressure $Paw_{PEEP}$ and esophageal pressure $Pes_{PEEP}$ in a state where the positive expiratory end pressure is non-zero, and acquires the airway pressure $Paw_{ZEEP}$ and esophageal pressure $Pes_{ZEEP}$ in a state where the positive expiratory end pressure is zero. The processor 50 adds the transpulmonary pressure with ($Paw_{PEEP}-Paw_{ZEEP}$) and subtracts ($Pes_{PEEP}-Pes_{ZEEP}$) from the sum, to obtain the corrected transpulmonary pressure.

In some embodiments, the processor 50 also corrects the transpulmonary pressure value by lung compliance and chest wall compliance. Specifically, the processor 50 acquires the lung compliance Clung and the chest wall compliance Ccw. It should be noted that there are many methods for processor 50 to obtain the lung compliance Clung and the chest wall compliance Ccw. For example, the processor 50 can obtain the chest wall compliance Ccw through the following formula:

$$Ccw = \frac{TV}{PesI-PEEP_{es}}.$$

Wherein, TV is tidal volume, PesI is esophageal pressure at inspiratory end, $PEEP_{es}$ is esophageal pressure at expiratory end. Then the total compliance $C_{state}$ can be calculated through the following formula:

$$C_{state} = \frac{\text{TV}}{\text{Pplat} - \text{PEEP}}.$$

Wherein, TV is tidal volume, Pplat is plateau pressure, PEEP is positive expiratory end pressure. When the total compliance $C_{state}$ and the chest wall compliance Ccw are calculated, the lung compliance Clung can be calculated by solving the following equation:

$$\frac{1}{\text{Clung}} + \frac{1}{\text{Ccw}} = \frac{1}{C_{state}}.$$

After acquiring the lung compliance Clung and the chest wall compliance Ccw, the processor 50 can calculate an error compensation value through the following formula:

$$\Delta \text{Ptrans}_{erro} = \text{Ptrans} - \text{Plung} * \frac{\frac{1}{\text{Clung}}}{\frac{1}{\text{Clung}} + \frac{1}{\text{Ccw}}}.$$

Wherein, $\Delta \text{Ptrans}_{erro}$ is the error compensation value, Ptrans is the transpulmonary pressure value, Plung is the intrapulmonary pressure value.

The processor 50 subtracts the error compensation value from the transpulmonary pressure value to obtain the corrected transpulmonary pressure value.

In some embodiments, the processor 50 may calculate a transdiaphragmatic pressure by subtracting either intraabdominal pressure or intragastric pressure from either intrathoracic pressure or esophageal pressure. For example, the transdiaphragmatic pressure can be obtained by subtracting the intragastric pressure from the esophageal pressure. It should be noted that in some embodiments, if the pressure sensor is inserted into the abdomen through a wound incision, the intraabdominal pressure can be acquired. In some embodiments, the processor 50 may also correct the transdiaphragmatic pressure. For example, the processor 50 acquires the esophageal pressure $\text{Pes}_{PEEP}$ and the intragastric pressure $\text{Psto}_{PEEP}$ when the positive expiratory end pressure is non-zero, and acquires the esophageal pressure $\text{Pes}_{ZEEP}$ and the intragastric pressure $\text{Psto}_{ZEEP}$ when the positive expiratory end pressure is zero. The processor 50 adds the transdiaphragmatic pressure with $(\text{Pes}_{PEEP}-\text{Pes}_{ZEEP})$ and subtracts $(\text{Psto}_{PEEP}-\text{Psto}_{ZEEP})$ from the sum, to obtain the corrected transdiaphragmatic pressure.

The above are some descriptions of the airway pressure, intrathoracic pressure, carinal pressure, intrapulmonary pressure, esophageal pressure, intragastric pressure, intraabdominal pressure, transpulmonary pressure and transdiaphragmatic pressure.

In this disclosure, the processor 50 receives signals of the pressure sensor 30 and the flow sensor 10, and calculates the energy which acts on the respiratory system of the patient during the mechanical ventilation process according to acquired pressure and gas flow rate. In some embodiments, the processor 50 integrates the acquired pressure and gas flow rate to obtain the energy which acts on the respiratory system of the patient during the mechanical ventilation process. In some embodiments, the processor 50 integrates the acquired pressure and gas flow rate within a preset unit time, such as 1 minute, to obtain the energy which acts on the respiratory system of the patient during the mechanical ventilation process. In some embodiments, the processor 50 integrates the acquired pressure and gas flow rate in a respiratory cycle and multiplies the integral value by the respiratory rate to obtain the energy which acts on the respiratory system of the patient during mechanical ventilation process. The following is a further explanation of how to calculate the energy which acts on the respiratory system of the patient during mechanical ventilation process in combination with the pressure at different points of the respiratory system.

In some embodiments, the processor 50 calculates the energy which acts on the respiratory system of the patient during the mechanical ventilation process according to the airway pressure and the gas flow rate. For example, the energy which acts on the respiratory system of the patient during the mechanical ventilation process can be obtained by integrating the airway pressure and gas flow rate. The formula is as follows:

$$\text{Energy}_{rs} = \int_{0}^{\text{Tinsp}} \text{Paw} * \text{Flow dt}.$$

Wherein $\text{Energy}_{rs}$ represents energy which acts on the respiratory system of the patient during the mechanical ventilation process and is obtained by integrating the airway pressure and gas flow rate of a signal cycle, Tinsp represents inspiratory time for each respiratory cycle, Paw represents airway pressure, and Flow represents gas flow rate. Of course, the energy which is calculated in a single cycle can also be combined with respiratory rate to obtain energy of each minute, and the formula is as follows:

$$\text{Power}_{rs} = 0.098 * \text{RR} * \int_0^{\text{Tinsp}} \text{Paw} * \text{Flow dt}.$$

Wherein, unit of airway pressure Paw is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the airway pressure and gas flow rate $\text{Power}_{rs}$ is J/min. As $1cmH_2O*1L/min = 0.098J/min$, there is a coefficient of 0.098 in the above formula. Of course, the energy $\text{Energy}_{rs}$ of all cycles within 1 minute can be directly accumulated to obtain the energy per minute.

In some embodiments, when calculating the energy which acts on the respiratory system of the patient during the mechanical ventilation process according to the airway pressure and gas flow rate, potential energy which is generated by the tidal volume part formed by positive expiratory end pressure can also be considered. This part of energy is generally a fixed value and never changes with the mechanical ventilation process, and it can often be omitted because of the need for additional positive expiratory end pressure release. When considering this part of potential energy, the above formula becomes:

$$\text{Energy}_{rs} = \int_0^{\text{Tinsp}} \text{Paw} * \text{Flow dt} + \frac{1}{2} * \text{PEEP}_{\text{volume}} * \text{PEEP}.$$

Then energy per minute is obtained after unit conversion combined with the respiratory rate:

$$\text{Power}_{rs} = 0.098 * \text{RR} * \left( \int_0^{\text{Tinsp}} \text{Paw} * \text{Flow dt} + \frac{1}{2} * \text{PEEP}_{\text{volume}} * \text{PEEP} \right).$$

In these two formulas, $\text{PEEP}_{\text{Volume}}$ represents tidal volume which is caused by positive expiratory end pressure and has unit L, specifically, represents volume exhaled when the positive expiratory end pressure drops to zero; PEEP represents positive expiratory end pressure.

The energy which acts on the respiratory system of the patient during the mechanical ventilation process and is calculated according to the airway pressure and gas flow rate, can represent the energy which acts on the whole respiratory system of the patient during the mechanical ventilation process, such as the total energy which acts on trachea, chest wall and lungs of the patient.

In some embodiments, the processor 50 calculates the energy which acts on the respiratory system of the patient during the mechanical ventilation process according to values of intrapulmonary pressure and gas flow rate. It should be noted that the intrapulmonary pressure can be acquired by the pressure sensor 10 or estimated through the airway pressure, which has been described in detail above and is repeated here. In some examples, the energy which acts on the respiratory system of the patient during the mechanical ventilation process is obtained by integrating the intrapulmonary pressure and gas flow rate. The formula is as follows:

$$\text{Energy}_{\text{lung}} = \int_0^{\text{Tinsp}} \text{Plung} * \text{Flow dt}.$$

Wherein $\text{Energy}_{\text{lung}}$ represents energy which acts on the respiratory system of the patient during the mechanical ventilation process and is obtained by integrating the intrapulmonary pressure and gas flow rate of a signal cycle, Tinsp represents inspiratory time for each respiratory cycle, Plung represents intrapulmonary pressure, and Flow represents gas flow rate. Of course, the energy which is calculated in a single cycle can also be combined with respiratory rate to obtain energy of each minute, and the formula is as follows:

$$\text{Power}_{\text{lung}} = 0.098 * \text{RR} * \int_0^{\text{Tinsp}} \text{Plung} * \text{Flow dt}.$$

Wherein, unit of intrapulmonary pressure Plung is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the intrapulmonary pressure and gas flow rate $\text{Power}_{\text{lung}}$ is J/min. As $1cmH_2O*1L/min = 0.098J/min$, there is a coefficient of 0.098 in the above formula. Of course, the energy per second or hour can be calculated according to specific needs. At this time, the corresponding unit is J/s or J/h. Correspondingly, the coefficient 0.098 in the above formula corresponds to other values which are converted according to units.

In some embodiments, when calculating the energy which acts on the respiratory system of the patient during the mechanical ventilation process according to intrapulmonary pressure and gas flow rate, potential energy which is generated by the tidal volume part formed by intrapulmonary pressure at expiratory end can also be considered. This part of energy is generally a fixed value and never changes with the mechanical ventilation process, and it can often be omitted. When considering this part of potential energy, the above formula becomes:

$$\text{Energy}_{\text{lung}} = \int_0^{\text{Tinsp}} \text{Plung} * \text{Flow dt} + \frac{1}{2} * \text{PlungE}_{\text{volume}} * \text{PlungE}.$$

Then energy per minute is obtained after unit conversion combined with the respiratory rate:

$$\text{Power}_{\text{lung}} =$$

$$0.098 * \text{RR} * \left( \int_0^{\text{Tinsp}} \text{Plung} * \text{Flow dt} + \frac{1}{2} * \text{PlungE}_{\text{volume}} * \text{PlungE} \right).$$

In these two formulas, $\text{PlungE}_{\text{Volume}}$ represents tidal volume which is caused by intrapulmonary pressure at expiratory end and has unit L, specifically, represents volume exhaled when the intrapulmonary pressure at expiratory end drops to zero; PlungE represents intrapulmonary pressure at expiratory end.

The energy which acts on the respiratory system of the patient during the mechanical ventilation process and is calculated according to the intrapulmonary pressure and gas flow rate, can represent the energy which acts on the chest wall and lungs of the patient during the mechanical ventilation process.

In some embodiments, the processor 50 calculates the energy which acts on the respiratory system of the patient during the mechanical ventilation process according to values of transpulmonary pressure and gas flow rate. For example, the energy which acts on the respiratory system of the patient during the mechanical ventilation process is obtained by integrating the transpulmonary pressure and gas flow rate. The formula is as follows:

$$\text{Energy}_{\text{tr}} = \int_0^{\text{Tinsp}} \text{Ptrans} * \text{Flow dt}.$$

Wherein, $\text{Energy}_{\text{tr}}$ represents energy which acts on the respiratory system of the patient during the mechanical ventilation process and is obtained by integrating the transpulmonary pressure and gas flow rate of a signal cycle, Tinsp represents inspiratory time for each respiratory cycle, Ptrans represents transpulmonary pressure, and Flow represents gas flow rate. Of course, the energy which is calculated in a single cycle can also be combined with respiratory rate to obtain energy of each minute, and the formula is as follows:

$$\text{Power}_{\text{tr}} = 0.098 * \text{RR} * \int_0^{\text{Tinsp}} \text{Ptrans} * \text{Flow dt}.$$

Wherein, unit of transpulmonary pressure Ptrans is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the transpulmonary pressure and gas flow rate $\text{Power}_{\text{tr}}$ is J/min. As $1cmH_2O*1L/min = 0.098J/min$, there is a coefficient of 0.098 in the above formula. Similarly, the unit and coefficient can also be set as required.

In some embodiments, when calculating the energy which acts on the respiratory system of the patient during the mechanical ventilation process according to transpulmonary pressure and gas flow rate, potential energy which is generated by the tidal volume part formed by transpulmonary pressure at expiratory end can also be considered. This part of energy is generally a fixed value and never changes with the mechanical ventilation process, and it can often be omitted. When considering this part of potential energy, the above formula becomes:

$$\text{Energy}_{\text{tr}} = \int_0^{\text{Tinsp}} \text{Ptrans} * \text{Flow dt} + \frac{1}{2} * \text{PtansE}_{\text{volume}} * \text{PtransE}.$$

Then energy per minute is obtained after unit conversion combined with the respiratory rate:

$$Power_{tr} =$$
$$0.098 * RR * \left( \int_0^{Tinsp} Ptrans * Flow\ dt + \frac{1}{2} * PtansE_{volume} * PtransE \right).$$

In these two formulas, $PtansE_{volume}$ represents tidal volume which is caused by transpulmonary pressure at expiratory end and has unit L, specifically, represents volume exhaled when the transpulmonary pressure at expiratory end drops to zero; PtransE represents transpulmonary pressure at expiratory end.

The energy which acts on the respiratory system of the patient during the mechanical ventilation process and is calculated according to the transpulmonary pressure and gas flow rate, can represent the energy of the mechanical ventilation process which acts on the lungs of the patient.

In some embodiments, the processor 50 calculates the energy which acts on the respiratory system of the patient during the mechanical ventilation process according to values of transdiaphragmatic pressure and gas flow rate. For example, the energy which acts on the respiratory system of the patient during the mechanical ventilation process is obtained by integrating the transdiaphragmatic pressure and gas flow rate. The formula is as follows:

$$Energy_{di} = \int_0^{Tinsp} Pdi * Flow\ dt;$$

Wherein, $Energy_{di}$ represents energy which acts on the respiratory system of the patient during the mechanical ventilation process and is obtained by integrating the transdiaphragmatic pressure and gas flow rate of a signal cycle, Tinsp represents inspiratory time for each respiratory cycle, Pdi represents transdiaphragmatic pressure, and Flow represents gas flow rate. Of course, the energy which is calculated in a single cycle can also be combined with respiratory rate to obtain energy of each minute, and the formula is as follows:

$$Power_{di} = 0.098 * RR * \int_0^{Tinsp} Pdi * Flow\ dt.$$

Wherein, unit of transdiaphragmatic pressure Pdi is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the transdiaphragmatic pressure and gas flow rate $Power_{di}$ is J/min. As $1cmH_2O*1L/min = 0.098J/min$, there is a coefficient of 0.098 in the above formula. Similarly, the unit and coefficient can also be set as required.

In some embodiments, when calculating the energy which acts on the respiratory system of the patient during the mechanical ventilation process according to transdiaphragmatic pressure and gas flow rate, potential energy which is generated by the tidal volume part formed by transdiaphragmatic pressure at expiratory end can also be considered. This part of energy is generally a fixed value and never changes with the mechanical ventilation process, and it can often be omitted. When considering this part of potential energy, the above formula becomes:

$$Energyr_{di} = \int_0^{Tinsp} Pdi * Flow\ dt + \frac{1}{2} * PdiE_{volume} * PdiE.$$

Then energy per minute is obtained after unit conversion combined with the respiratory rate:

$$Power_{di} =$$
$$0.098 * RR * \left( \int_0^{Tinsp} Pdi * Flow\ dt + \frac{1}{2} * PdiE_{volume} * PdiE \right).$$

In these two formulas, $PdiE_{volume}$ represents tidal volume which is caused by transdiaphragmatic pressure at expiratory end and has unit L, specifically, represents volume exhaled when the transdiaphragmatic pressure at expiratory end drops to zero; PdiE represents transdiaphragmatic pressure at expiratory end.

The energy which acts on the respiratory system of the patient during the mechanical ventilation process and is calculated according to the transdiaphragmatic pressure and gas flow rate, can represent the energy of the mechanical ventilation process which acts on the diaphragm of the patient.

The above is some explanation about calculating the energy which acts on the respiratory system of the patient during the mechanical ventilation process.

Lung injury VILI is a combination of various types of injury and may include one or more of barotrauma, volumetric injury, atelectasis injury, tissue injury, etc. As mentioned above, the energy which acts on the respiratory system of the patient during the mechanical ventilation process can evaluate the lung injury more accurately, truly and in real time, but it is a relatively comprehensive description of the lung injury. In fact, the role of each ventilation parameter in the mechanical ventilation process is particularly important, and each ventilation parameter affects the role of each part of the energy which acts on the respiratory system of the patient during the mechanical ventilation process. Therefore, in some embodiments, the apparatus for monitoring ventilation of a patient also determines a degree of contribution of ventilation parameter to the energy which acts on the respiratory system of the patient during the mechanical ventilation process, and these are described in detail below.

In some embodiments, the processor 50 acquires a ventilation parameter and determines a degree of contribution of the ventilation parameter to the energy which acts on the respiratory system of the patient during the mechanical ventilation process. In some embodiments, the ventilation parameter includes a ventilation control parameter and/or a respiratory system related parameter.

In some embodiments, the ventilation control parameter may include one or more of tidal volume, gas flow rate, drive pressure, positive respiratory end pressure, respiratory rate, and respiratory ratio. When determining that the degree of contribution of the ventilation parameter to the energy which acts on the respiratory system of the patient during the mechanical ventilation process, the degree of contribution of the ventilation parameter to the energy which acts on the respiratory system of the patient during the mechanical ventilation process, can be determined according to the change of the energy, which is caused by changing the value of the ventilation parameter.

Degrees of contribution can be reflected in different ways:

1. Monitored value of mechanical energy which is generated by the ventilation parameter, such as mechanical energy PowerR, which is generated by respiratory resistance;
2. Proportion of the mechanical energy which is generated by the ventilation parameter, that is, the proportion of the mechanical energy which is generated by each ventilation parameter in the total mechanical energy, for example, the proportion of the mechanical energy which is generated by respiratory resistance in the total mechanical energy, PowerR/Powerrs; or
3. Rate of change of the mechanical energy which is generated by the ventilation parameter, such as a ratio of increase percentage of corresponding ventilation parameter to increase percentage of corresponding mechanical energy.

Specifically, when determining that a degree of contribution of any one ventilation control parameter, other ventilation control parameters can be kept unchanged, and then the change relationship between the ventilation control parameter of which the degree of contribution is to be determined and the energy which acts on the respiratory system of the patient during the mechanical ventilation process, can be determined. For example, for each change of a fixed amount of the ventilation control parameter of which the degree of contribution is to be determined, corresponding change amount of the energy which acts on the respiratory system of the patient during the mechanical ventilation process is obtained, and then the relationship between the ventilation control parameter of which the degree of contribution is to be determined and the energy which acts on the respiratory system of the patient during the mechanical ventilation process is obtained through fitting, such as linear fitting or exponential fitting. In this way, the degree of contribution of the ventilation control parameter can be determined. Of course, it is also possible to control the change of a certain ventilation control parameter without by a fixed amount. Take the mechanical ventilation process under the volume mode as an example. The ventilation control parameters involved in this mode usually include tidal volume, gas flow rate, positive respiratory end pressure, respiratory rate and respiratory ratio. In order to calculate the degree of contribution of tidal volume to the energy which acts on the respiratory system of the patient during the mechanical ventilation process, the gas flow rate, positive respiratory end pressure, respiratory rate and respiratory ratio can be maintained as fixed values, and then the value of tidal volume can be changed, for example, each time the tidal volume is increased by a fixed percentage X% -- for example, 2% -- and then the increased percentage Y% -- for example, Y1%, Y2%,..., Yn% of the energy which acts on the respiratory system of the patient in the mechanical ventilation process is obtained. Then the relationship between X and Y is obtained by fitting. For example, assuming that K= Y/X, then the degree of contribution of tidal volume to the energy which acts on the respiratory system of the patient in the mechanical ventilation process is K. For example, the following is the table of changes in ventilation parameters and energy which acts on the respiratory system of the patient during the mechanical ventilation process, i.e., Table 1 below. In the table, Power represents the energy which acts on the respiratory system of the patient during the mechanical ventilation process, TV represents tidal volume, Rate represents respiratory rate, I represents inspiratory time, and E represents expiratory time. It can be seen that I: E in the table is 1/2. Raw represents respiratory system resistance, Crs represents respiratory system compliance, and PEEP represents positive airway pressure at expiratory end. The change from state 1 to state 2 is because TV in ventilation parameters changes from 500 to 1000, i.e., an increase of 100%. Correspondingly, Power increases from 5.82 to 17.4, an increase of approximately 200%. If linear fitting is performed, the rate of change or degree of contribution of TV is 2. The change from state 1 to state 3 is because the change of Rate in ventilation parameters is from 12 to 24, i.e., an increase of 100%. Correspondingly, Power increases from 5.82 to 14.46, an increase of approximately 150%. If linear fitting is performed, the rate of change or degree of contribution of Rate is 1.5.

Table 1

| Parameter | Unit | State 1 | State 2 | State 3 |
|-----------|------|---------|---------|---------|
| POWER | J/min | 5.82 | 17.4 | 14.46 |
| TV | mL | 500 | 1000 | 500 |
| Rate | bpm | 12 | 12 | 24 |
| I | | 1 | 1 | 1 |
| E | | 2 | 2 | 2 |
| Raw | cmH2O/L/s | 8 | 8 | 8 |
| Crs | mL/cmH2O | 100 | 100 | 100 |
| PEEP | cmH2O | 5 | 5 | 5 |

It can be understood that when the gas flow rate, positive respiratory end pressure, respiratory rate and respiratory ratio are maintained as fixed values, these fixed values affect the value of the degree of contribution of tidal volume. For example, when the gas flow rate takes two different fixed values, the degrees of contribution of tidal volume are calculated separately, and the obtained degrees of contributions of tidal volume are generally different. However, a ranking of degree of contribution of these ventilation control parameters to the energy which acts on the respiratory system of the patient during the mechanical ventilation process is generally fixed.

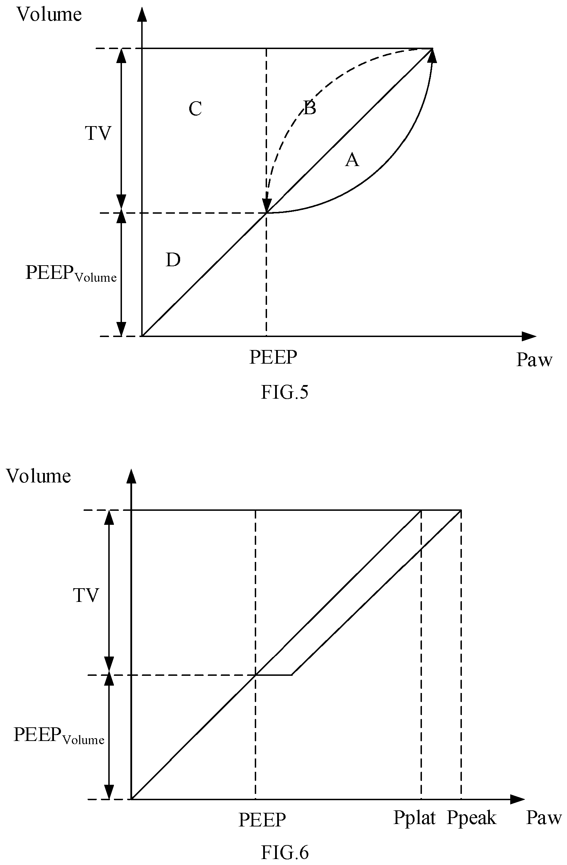

In some embodiments, the respiratory system related parameters may include one or more of respiratory system compliance and respiratory system resistance of the patient. The degree of contribution of respiratory system compliance to the energy which acts on the respiratory system of the patient during the mechanical ventilation process can be expressed as proportion of work which is done to overcome the respiratory system compliance in the energy which acts on the respiratory system of the patient during the mechanical ventilation process. Similarly, the degree of contribution of respiratory system resistance to the energy which acts on the respiratory system of the patient during the mechanical ventilation process can be expressed as the proportion of work which is done to overcome the respiratory system resistance in the energy which acts on the respiratory system of the patient during the mechanical ventilation process. The following is a detailed description. Please refer to FIG. 5 for the static pressure-volume curve (P-V curve) of the respiratory system, which can reflect the mechanical state of the respiratory system of the patient. The abscissa shown in the figure is the airway pressure Paw. In some other examples, the abscissa may also be the intrapulmonary pressure Plung, the transpulmonary pressure Ptrans, or the transdiaphragmatic pressure Pdi.

The abscissa of airway pressure Paw is taken for example. In the figure, A is kinetic energy part, which indicates the amount of work which is done to overcome the respiratory system resistance. In the figure, B, C and D are potential energy parts, which are related to the respiratory system compliance. Specifically, A and B are dynamically changing, and they work with the changes of tidal volume and drive pressure. While C and D are static. When the drive pressure changes above the positive expiratory end pressure PEEP, the tidal volume and pressure which are related to the positive expiratory end pressure PEEP do not work, but there is still elastic potential energy, which is part of the energy which acts on the respiratory system of the patient during the mechanical ventilation process.

In one example, the potential energy part which is related to the respiratory system compliance in the figure, i.e., B, C and D, can be calculated by the following formula:

$$\text{Energy}_C = \int_0^{\text{Tinsp}} \text{Plung} * \text{Flow dt} + \frac{1}{2} * \text{PEEP}_{\text{volume}} * \text{PEEP}.$$

Wherein Energy$_C$ represents energy which is related to the respiratory system compliance in a single cycle, Tinsp represents inspiratory time for each respiratory cycle, Plung represents intrapulmonary pressure, and Flow represents gas flow rate; PEEP$_{\text{Volume}}$ represents tidal volume which is caused by positive expiratory end pressure and has unit L, specifically, represents volume exhaled when the positive expiratory end pressure drops to zero; PEEP represents positive expiratory end pressure.

In some examples, the potential energy part which is related to the respiratory system compliance may be further divided into the potential energy part which is related to respiratory system dynamic compliance and the potential energy part which is related to respiratory system static compliance. Specifically, the work which is done to overcome the respiratory system dynamic compliance in the figure, that is, the potential energy parts of B and C in the figure are:

$$Energy_{Cdyn} = \int_0^{Tinsp} Plung * Flow\ dt.$$

Wherein $Energy_{Cdyn}$ represents energy which is related to the respiratory system dynamic compliance in a single cycle, that is, the work which is done to overcome the respiratory system dynamic compliance, Tinsp represents inspiratory time for each respiratory cycle, Plung represents intrapulmonary pressure, and Flow represents gas flow rate.

The work which is done to overcome the respiratory system static compliance in the figure, that is, the potential energy part of D in the figure is:

$$Energy_{Cstat} = \frac{1}{2} * PEEP_{volume} * PEEP.$$

Wherein $Energy_{Cstat}$ represents energy which is related to the respiratory system static compliance in a single cycle, that is, the work which is done to overcome the respiratory system static compliance; $PEEP_{Volume}$ represents tidal volume which is caused by positive expiratory end pressure and has unit L, specifically, represents volume exhaled when the positive expiratory end pressure drops to zero; PEEP represents positive expiratory end pressure.

In one example, the work done in the figure to overcome the respiratory system resistance, that is, the kinetic energy part A, can be calculated by the following formula:

$$Energy_R = \int_0^{Tinsp} (Paw - Plung) * Flow\ dt.$$

Wherein $Energy_R$ represents work which is done to overcome the respiratory system resistance in a single cycle, Tinsp represents inspiratory time for each respiratory cycle, Paw represents airway pressure, Plung represents intrapulmonary pressure, and Flow represents gas flow rate.

Of course, the energy which is calculated in a single cycle can also be combined with respiratory rate to obtain energy of each minute. The energy $Energy_C$, $Energy_{Cdyn}$, $Energy_{Cstat}$ and $Energy_R$ mentioned above are described below.

The conversion of energy $Energy_C$ which is related to respiratory system compliance in a single cycle is as follows:

$$Power_C = 0.098 * RR * \left( \int_0^{Tinsp} Plung * Flow\ dt + \frac{1}{2} * PEEP_{volume} * PEEP \right).$$

The conversion of energy $Energy_{Cdyn}$ which is related to respiratory system dynamic compliance in a single cycle is as follows:

$$Power_{Cdyn} = 0.098 * RR * \left( \int_0^{Tinsp} Plung * Flow\ dt \right).$$

The conversion of energy $Energy_{Cstat}$ which is related to respiratory system static compliance in a single cycle is as follows:

$$Power_{Cstat} = 0.098 * RR * \left( \frac{1}{2} * PEEP_{volume} * PEEP \right).$$

The conversion of work $Energy_R$ which is done to overcome the respiratory system resistance in a single cycle is as follows:

$$Power_R = 0.098 * RR * \left( \int_0^{Tinsp} (Paw - Plung) * Flow\ dt \right).$$

In the above formula, units of airway pressure Paw and intrapulmonary pressure Plung are $cmH_2O$, unit of gas flow rate Flow is L/min; unit of inspiratory time for each respiratory cycle Tinsp is s, RR represents respiratory rate, and its unit is per minute. Units of energy $Power_C$ which is related to respiratory system compliance for each minute, energy $Power_{Cdyn}$ which is related to respiratory system dynamic compliance for each minute, energy $Power_{Cstat}$ which is related to respiratory system static compliance for each minute and work $Power_R$ which is done to overcome the respiratory system resistance for each minute are J/min. As $1cmH_2O*1L/min = 0.098J/min$, there is a coefficient of 0.098 in the above formula. As $1cmH_2O*1L/min = 0.098J/min$, there is a coefficient of 0.098 in the above formula.

Of course, the energy $Energy_C$ of all cycles within 1 minute can be directly accumulated to obtain the energy per minute $Power_C$; the energy $Energy_{Cdyn}$ of all cycles within 1 minute can be directly accumulated to obtain the energy per minute $Power_{Cdyn}$; the energy $Energy_{Cstat}$ of all cycles within 1 minute can be directly accumulated to obtain the energy per minute $Power_{Cstat}$; the work $Energy_R$ of all cycles within 1 minute can be directly accumulated to obtain the work per minute $Power_R$.

The above is to calculate the degree of contribution of the respiratory system related parameters to the energy which acts on the respiratory system of the patient during the mechanical ventilation process by the integral method. In some embodiments, the degree of contribution of the respiratory system related parameters to the energy which acts on the respiratory system of the patient during the mechanical ventilation process can also be calculated by the formula method.

Similar to FIG.5, FIG.6 is also the static pressure-volume curve (P-V curve) of the respiratory system. The abscissa of airway pressure Paw is taken for example in FIG.6. The energy which acts on the respiratory system of the patient during the mechanical ventilation process is as follows:

$$\text{Energy}_{rs} = \left[TV * \left(\frac{500*TV}{Crs} + RR * \frac{(1+I:E)*TV*Raw}{60*I:E}\right) + TV * PEEP\right].$$

Wherein $Energy_{rs}$ represents energy which acts on the respiratory system of the patient during the mechanical ventilation process of a signal cycle, TV represents tidal volume, Crs represents respiratory system compliance, RR represents respiratory rate, I: E represents a ratio of inspiratory time and expiratory time, that is the respiratory ratio; Raw represents respiratory system resistance, PEEP represents positive expiratory end pressure. Of course, the energy which is calculated in a single cycle can also be combined with respiratory rate to obtain energy of each minute, and the formula is as follows:

$$\text{Power}_{rs} = 0.098 * RR * \left[TV * \left(\frac{500*TV}{Crs} + RR * \frac{(1+I:E)*TV*Raw}{60*I:E}\right) + TV * PEEP\right].$$

Wherein unit of energy for each minute $Power_{rs}$ is J/min; unit of respiratory rate RR is per minute; unit of tidal volume TV is L; unit of respiratory system compliance Crs is $ml/cmH_2O$; unit of respiratory system resistance Raw is $cmH_2O/L/s$; unit of positive expiratory end pressure PEEP is $cmH_2O$.

In general, when calculating the energy which acts on the respiratory system of the patient during the mechanical ventilation process, potential energy which is generated by the tidal volume part formed by positive expiratory end pressure can also be considered. This part of energy is generally a fixed value and never changes with the mechanical ventilation process, and it can often be omitted because of the need for additional positive expiratory end pressure release. When considering this part of potential energy, the above formula becomes:

$$\text{Energy}_{rs} = \left[TV * \left(\frac{500*TV}{Crs} + RR * \frac{(1+I:E)*TV*Raw}{60*I:E}\right) + TV * PEEP + \frac{1}{2} * PEEP_{volume} * PEEP\right].$$

Then energy per minute is obtained after unit conversion combined with the respiratory rate:

$$\text{Power}_{rs} = 0.098 * RR * \left[TV * \left(\frac{500*TV}{Crs} + RR * \frac{(1+I:E)*TV*Raw}{60*I:E}\right) + TV * PEEP + \frac{1}{2} * PEEP_{volume} * PEEP\right].$$

In these two formulas, $PEEP_{Volume}$ represents tidal volume which is caused by positive expiratory end pressure and has unit L, specifically, represents volume exhaled when the positive expiratory end pressure drops to zero.

The degree of contribution of respiratory system related parameter is calculated below. Taking energy of a single cycle which acts on the respiratory system of the patient during the mechanical ventilation process as

$$\text{Energy}_{rs} = [\text{TV} * \left(\frac{500*\text{TV}}{\text{Crs}} + RR * \frac{(1+\text{I:E})*\text{TV}*\text{Raw}}{60*\text{I:E}}\right) + \text{TV} * PEEP]$$ , and energy of each minute

as $$\text{Power}_{rs} = 0.098 * RR * [\text{TV} * \left(\frac{500*\text{TV}}{\text{Crs}} + RR * \frac{(1+\text{I:E})*\text{TV}*\text{Raw}}{60*\text{I:E}}\right) + \text{TV} * PEEP]$$ for ex-

ample, it can be seen from the respiratory mechanics equation that the following equation holds:

$$\left(\frac{\text{TV}*1000}{\text{Crs}}\right) = P_{plat} - PEEP;$$

$$RR * \frac{(1+\text{I:E})\text{TV}}{60*\text{I:E}} = \frac{\text{TV}}{\text{Tinsp}} = \text{Flow};$$

$$\text{Flow} * \text{Raw} = \left(\text{Ppeak} - P_{plat}\right).$$

Wherein, Pplat is plateau pressure, Tinsp represents inspiratory time for each respiratory cycle, Ppeak represents peak pressure shown in the figure, Flow represents gas flow rate. Therefore, it can be deduced that:

$$\text{Energy}_{rs} = [\frac{\text{TV}*(P_{plat}-PEEP)}{2} + \text{TV} * \left(\text{Ppeak} - P_{plat}\right) + \text{TV} * PEEP];$$

$$\text{Pawer}_{rs} = 0.098 * RR * [\frac{\text{TV}*(P_{plat}-PEEP)}{2} + \text{TV} * \left(\text{Ppeak} - P_{plat}\right) + \text{TV} * PEEP].$$

Therefore, the work which is done to overcome the respiratory system resistance is:

$$\text{Energy}_{R} = \text{TV} * \left(\text{Ppeak} - \text{Pplat}\right) = TV * RR * \frac{(1+\text{I:E})\text{TV}*\text{Raw}}{60*\text{I:E}}.$$

The work which is done to overcome the respiratory system dynamic compliance is:

$$\text{Energy}_{Cdyn} = \text{TV} * PEEP.$$

The work which is done to overcome the respiratory system static compliance is:

$$\text{Energy}_{Csta} = \frac{\text{TV}*(P_{plat}-PEEP)}{2} = \frac{500*\text{TV}*\text{TV}}{\text{Crs}}.$$

The work which is done to overcome the respiratory system compliance is equal to the work which is done to overcome the respiratory system dynamic compliance plus the work which is done to overcome the respiratory system static compliance.

Further, the ratio of the work which is done to overcome the respiratory system compliance to the energy which acts on the respiratory system of the patient during the mechanical ventilation process can also be calculated to represent the degree of contribution of the respiratory system compliance. Similarly, the ratio of the work which is done to overcome the respiratory system resistance to the energy which acts on the respiratory system of the patient during the mechanical ventilation process is calculated to represent the degree of contribution of the respiratory system resistance.

The above is some description of the degree of contribution of ventilation parameters, including ventilation control parameters and/or respiratory system related parameters, to the energy which acts on the respiratory system of the patient during the mechanical ventilation process. After determining the degree of contribution of ventilation parameters to the energy which acts on the respiratory system of the patient during the mechanical ventilation process, there are many ways to use the degree of contribution, which are described below.

In some embodiments, the display 70 may display the degree of contribution of each ventilation parameter in one or more ways such as word, number, character, table, diagram or icon. For example, the display 70 may display name of each ventilation parameter and value of its corresponding degree of contribution. In some specific embodiments, the display 70 can display the name of each ventilation parameter and its corresponding degree of contribution value in the order of ranking the degree of contribution from large to small or from small to large, so that medical personnel can easily understand the degree of contribution of each parameter to the energy which acts on the respiratory system of the patient during the mechanical ventilation process. For example, in an example of mechanical ventilation process under the volume mode, the display 70 may display the degree of contribution of ventilation control parameter involved in the mode, such as tidal volume, gas flow rate, positive respiratory end pressure, respiratory rate, and respiratory ratio. For another example, in an example of mechanical ventilation process under a pressure mode, the display 70 may display the degree of contribution of ventilation control parameter involved in the mode, such as tidal volume, drive pressure, positive respiratory end pressure, respiratory rate and respiratory ratio. Of course, in some examples, whether under the volume mode or the pressure mode, the display 70 may also display the degree of contribution of respiratory system related parameters such as the respiratory system compliance and respiratory system resistance of the patient. Therefore, in some examples, the display 70 can display the name of each ventilation control parameter and the value of its corresponding degree of contribution. Of course, in some examples, the display 70 may display the name of each ventilation control parameter and the ranking of its corresponding degree of contribution. In some other examples, the display 70 can graphically display the degree of contribution of each ventilation control parameter. In some other examples, the display 70 can display the name of each respiratory system related parameter and the value of its corresponding degree of contribution. In some other examples, the display 70 can display proportion of degrees of contribution of each respiratory system related parameter. In some other examples, the display 70 can graphically display the degree of contribution of each respiratory system related parameter. Of course, the display 70 may also display changes in the degree of contribution corresponding to each ventilation parameter by means of symbols or icons such as rising, falling, or parallel lines. In addition, the value of the degree of contribution can be reflected in various ways, such as monitored value, percentage or rate of change, and so on. Taking the respiratory system compliance as an example, displaying the value of its degree of contribution can be displaying the actual monitored value, percentage or rate of change of the work which is done to overcome the respiratory system compliance in the energy which acts on the respiratory system of the patient during the mechanical ventilation process. Similarly, displaying the value of the degree of contribution of the respiratory system resistance, may also be displaying the actual monitored value, percentage or rate of change of the work which is done to overcome the respiratory system resistance in the energy which acts on the respiratory system of the patient during the mechanical ventilation process. Table 2 below is an example.

Table 2

| Parameter | Unit | Normal patient | ARDS | COPD |
|---|---|---|---|---|
| TV | mL | 500 | 500 | 500 |
| Rate | bpm | 12 | 12 | 12 |
| I | | 1 | 1 | 1 |
| E | | 2 | 2 | 2 |
| Raw | cmH2O/L/s | 5 | 20 | 20 |
| Crs | mL/cmH2O | 50 | 20 | 100 |
| PEEP | cmH2O | 3 | 3 | 3 |
| Power | J/min | 5.59 | 12.64 | 6.76 |
| Powerc | J/min | 4.70 | 9.11 | 3.23 |
| $Power_R$ | J/min | 0.88 | 3.53 | 3.53 |
| $Power_C$% | | 84% | 72% | 48% |
| $Power_R$% | | 16% | 28% | 52% |

In the table, TV represents tidal volume, Rate represents respiratory rate, I represents inspiratory time, and E represents expiratory time. It can be seen that I: E in the table is 1/2. Raw represents respiratory system resistance, Crs represents respiratory system compliance, and PEEP represents positive expiratory end pressure. Power represents energy which acts on the respiratory system of the patient during the mechanical ventilation process. Powerc represents work, which is done to overcome the respiratory system compliance, and $Power_R$ represents work which is done to overcome the respiratory system resistance. Powerc% represents proportion of work, which is done to overcome the respiratory system compliance, i.e., the ratio of Powerc to Power; $Power_R$% represents proportion of work, which is done to overcome respiratory system resistance, i.e., the ratio of $Power_R$ to Power. It can be seen that Powerc% and $Power_R$ % of normal patients, patients with acute respiratory distress syndrome (ARDS) and patients with chronic obstructive pulmonary disease are different. In other words, Powerc% and $\text{Power}_R\%$ can give some information about diseases of the patient. Different patient types have different Powerc% and $\text{Power}_R\%$.

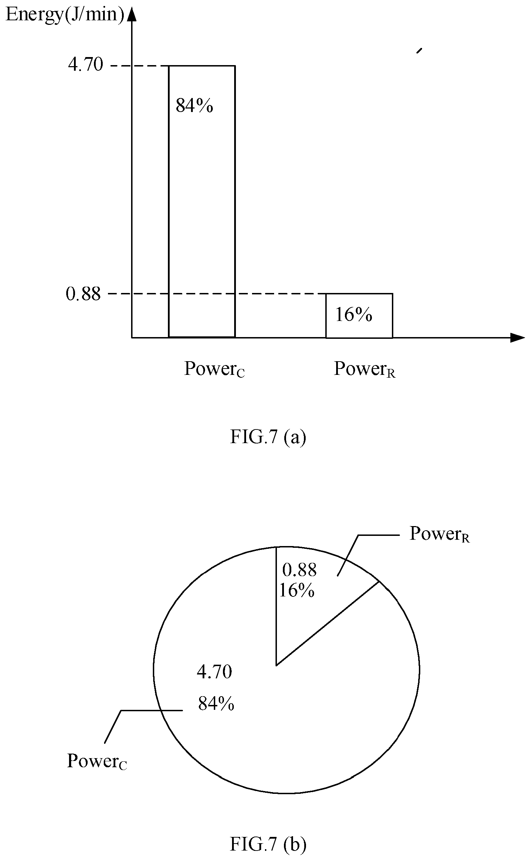

Therefore, in some embodiments, in addition to the above-mentioned display of the name of the ventilation parameter and the corresponding value of the degree of contribution, the degree of contribution of each ventilation parameter can also be displayed graphically, such as through a bar chart, a pie chart or a table. The above table shows the degree of contribution of respiratory system resistance (for example, the work which is done to overcome the respiratory system resistance and its proportion in the table) and the degree of contribution of the respiratory system compliance (for example, the work which is done to overcome the respiratory system compliance and its proportion in the table) graphically through a table. FIG. 7 (a) and FIG. 7 (b) show examples of the degree of contribution of the respiratory system resistance and the degree of contribution of the respiratory system compliance of the normal patient in bar charts and pie charts.

In some embodiments, the processor 50 may guide the mechanical ventilation process according to the degree of contribution of ventilation parameter. The following is a detailed description.

In some embodiments, when determining that the energy which acts on the respiratory system of the patient during the mechanical ventilation process, exceeds a first threshold, the processor 50 indicates to decrease the ventilation parameter according to a ranking of the degree of contribution or controls to decrease the ventilation parameter according to a ranking of the degree of contribution. Specifically, the processor 50 may indicate to preferentially decrease the ventilation parameter with the largest degree of contribution among the ventilation control parameters, or control to preferentially decrease the ventilation parameter with the largest degree of contribution among the ventilation control parameters. When determining that the energy which acts on the respiratory system of the patient during the mechanical ventilation process, exceeds a relatively safe threshold, the ventilation parameter with the largest degree of contribution among the ventilation control parameters can preferentially be manually or automatically decreased at this time, so that the energy which acts on the respiratory system of the patient during the mechanical ventilation process, can be quickly decreased to a safe range.

In some embodiments, when determining that the energy which acts on the respiratory system of the patient during the mechanical ventilation process, is lower than a second threshold, the processor 50 may indicate to increase the ventilation parameter according to a ranking of the degree of contribution or controls to increase the ventilation parameter according to a ranking of the degree of contribution. Specifically, the processor 50 may indicate to preferentially increase the ventilation parameter with the smallest degree of contribution among the ventilation control parameters, or control to preferentially increase the ventilation parameter with the smallest degree of contribution among the ventilation control parameters. When determining that the energy which acts on the respiratory system of the patient during the mechanical ventilation process, is lower than a relatively safe threshold, the ventilation parameter with the smallest degree of contribution among the ventilation control parameters can preferentially be manually or automatically increased, so that the energy which acts on the respiratory system of the patient during the mechanical ventilation process, can enter the safe range more smoothly and safely and would not exceed the standard amount and cause additional problems.

In some embodiments, the processor 50 estimates the energy which acts on the respiratory system of the patient during the mechanical ventilation process according to a setting command of the ventilation control parameter, and outputs the energy after setting the ventilation control parameter. Specifically, the estimated energy which acts on the respiratory system of the patient during the mechanical ventilation process may be displayed on the display 70 for medical personnel to view and make decisions. In some embodiments, the processor 50 may also determine whether to send an alarm according to the estimated energy which acts on the respiratory system of the patient during the mechanical ventilation process. For example, if the estimated energy which acts on the respiratory system of the patient during the mechanical ventilation process exceeds the first threshold or is lower than the second threshold, the processor 50 sends an alarm.

Some studies show that excessive energy which acts on the respiratory system of the patient during the mechanical ventilation process, has a significant clinical correlation with lung injury. Specifically, some clinical studies show that when the energy which acts on the whole respiratory system of the patient during the mechanical ventilation process is larger than 25J /min, or the energy which acts on the lungs of the patient during the mechanical ventilation process is larger than 12J /min or 13J/min and so on, it significantly cause lung injury. Some clinical studies show that when the energy which acts on the whole respiratory system of the patient during the mechanical ventilation process is larger than 17J /min, it significantly increase the mortality of patients. Therefore, the first threshold and the second threshold can be set according to the clinical data and the specific situation of the patient.

In some embodiments, the processor 50 can determine whether to send an alarm according to the degree of contribution of each respiratory system related parameter. For example, the processor 50 sends different alarms according to the proportion of the degree of contribution of the respiratory system resistance, that is, when the work which is done to overcome the respiratory system resistance accounts for the energy which acts on the respiratory system of the patient during the mechanical ventilation process in different ranges, the processor 50 sends different alarms. Similarly, the processor 50 sends different alarms according to the proportion of the degree of contribution of the respiratory system compliance, that is, when the work which is done to overcome the respiratory system compliance accounts for the energy which acts on the respiratory system of the patient during the mechanical ventilation process in different ranges, the processor 50 sends different alarms. The corresponding relationship between the specific proportion range and the specific alarm can be determined and preset by those skilled in the art according to the clinical data. For example, when the proportion of the degree of contribution of the respiratory system compliance is between 80% and 85%, no alarm is sent, and it is currently normal. When the proportion of the degree of contribution of the respiratory system compliance is between 70% and 75%, an alarm that the patient currently presents acute respiratory distress syndrome (ARDS) is sent. When the proportion of the degree of contribution of the respiratory system compliance is between 40% and 50%, an alarm that the patient is currently suffering from chronic obstructive pulmonary disease, is sent. Alternatively, when the degree of contribution of the respiratory system compliance is not in the range from 80% to 85%, an alarm is sent. Otherwise, no alarm is sent.

The above is description of the apparatus for monitoring ventilation of a patient in some embodiments of this disclosure. In some embodiments of this disclosure, a method for monitoring ventilation of a patient is also disclosed.

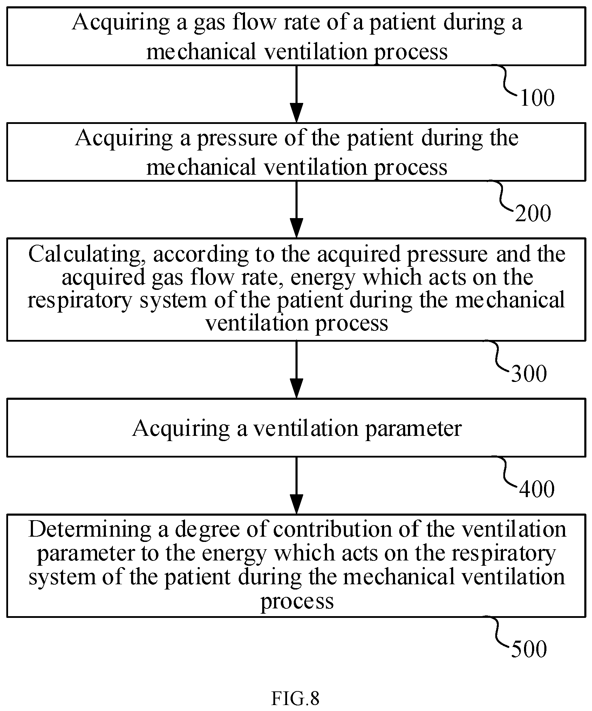

FIG. 8 is a flow diagram of a method for monitoring ventilation of a patient according to some embodiments of this disclosure, wherein the method includes following steps.

In step 100, a gas flow rate of a patient during a mechanical ventilation process is acquired.

The gas flow rate may refer to an inspiratory flow rate of the patient, or may refer to a flow rate during the inspiratory period and the expiratory period of the patient. The gas flow rate can be acquired by the flow sensor 30.

In step 200, a pressure of the patient during the mechanical ventilation process is acquired.

The pressure reflects a pressure which acts on different points of the respiratory system of the patient during the mechanical ventilation process, such as one or more of airway pressure, intrathoracic pressure, carinal pressure, intrapulmonary pressure, esophageal pressure and intragastric pressure. The various pressures described above can be acquired by the pressure sensor 10.

In some embodiments, the pressure sensor 10 can be a catheter-type pressure sensor or an optical fiber-type pressure sensor. By inserting the pressure sensor to the corresponding site of the respiratory system of the patient, the pressure at the corresponding site can be acquired. For example, if the pressure sensor is inserted into the airway of the patient, the airway pressure can be acquired. If the pressure sensor is inserted into the esophagus, the esophageal pressure can be acquired. If the pressure sensor is inserted into the stomach, the intragastric pressure can be acquired. If the pressure sensor is inserted into the carina inside the trachea, the carinal pressure can be acquired. If the pressure sensor is inserted into the stomach, the intragastric pressure can be acquired. If the pressure sensor is inserted into the thoracic cavity through a wound incision, the intrathoracic pressure can be acquired. In some embodiments, the pressures at some points in the respiratory system can also be used to replace or calculate the pressure at other points, which is illustrated by several examples below.

In some embodiments, the carinal pressure may be used to replace the intrapulmonary pressure. In some embodiments, the esophageal pressure may be used to replace the intrathoracic pressure. In some embodiments, the intragastric pressure may be used to replace the intraabdominal pressure.

In some embodiments, intrapulmonary pressure can be calculated according to the airway pressure in step 200. For example, in some embodiments, the intrapulmonary pressure is calculated according to the airway pressure, the respiratory system resistance, and the gas flow rate described above in step 200. In a specific example, it can be calculated by the following formula:

$$\text{Plung(t)}=\text{Paw(t)}-\text{Raw*Flow(t)}.$$

Wherein Plung(t) represents a function of intrapulmonary pressure which changes with time t, or represents a real-time intrapulmonary pressure. *Paw*(*t*) represents a function of airway pressure which changes with time t, or represents a real-time airway pressure. *Flow*(*t*) represents a function of the gas flow rate of the patient during the mechanical ventilation process which changes with time t, or represents a real-time gas flow rate of the patient during the mechanical ventilation process. Raw represents a respiratory system resistance.

In some embodiments, transpulmonary pressure can be calculated by subtracting either the esophageal pressure or the intrathoracic pressure from either the intrapulmonary pressure or the airway pressure in step 200. For example, the transpulmonary pressure is obtained by subtracting the esophageal pressure from the airway pressure.

In some embodiments, transdiaphragmatic pressure can be calculated by subtracting either intraabdominal pressure or intragastric pressure from either intrathoracic pressure or esophageal pressure in step 200. For example, the transdiaphragmatic pressure can be obtained by subtracting the intragastric pressure from the esophageal pressure. It should be noted that in some embodiments, if the pressure sensor is inserted into the abdomen through a wound incision, the intraabdominal pressure can be acquired.

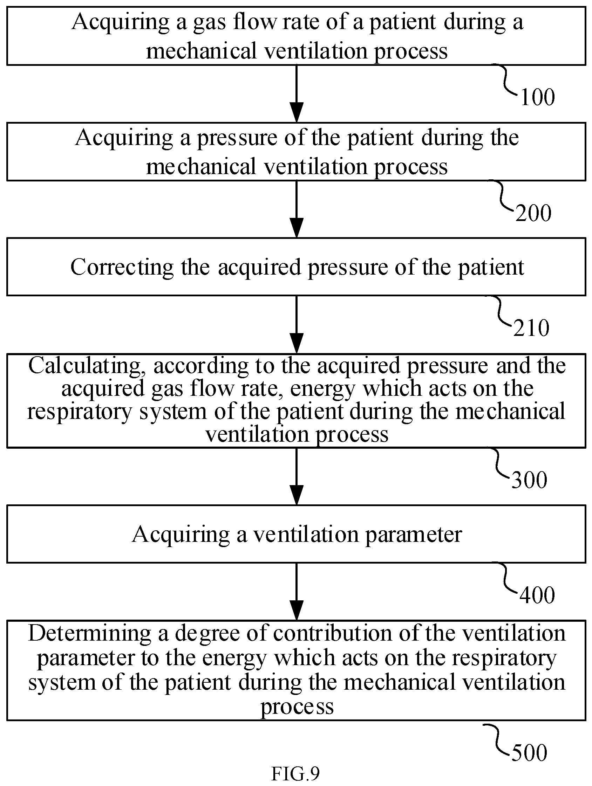

In some embodiments, in order to make the energy which acts on the respiratory system of the patient in the mechanical ventilation process and is calculated later more accurate, the acquired pressure of the patient can be corrected before calculation. FIG.9 is an example, and the pressure is corrected by introducing step 210.

In step 210, the acquired pressure of the patient is corrected. Step 210 may relate to correction of transpulmonary pressure or transdiaphragmatic pressure, which is described in detail below.

In some embodiments, after acquiring the transpulmonary pressure of the patient in step 200, the transpulmonary pressure is corrected. The followings are specific description for the correction method of the transpulmonary pressure in several examples.

In some embodiments, the transpulmonary pressure is corrected by the airway pressure value and the esophageal pressure value in the states where the positive expiratory end pressure is zero and non-zero in step 210. Specifically, in step 210; the airway pressure $Paw_{PEEP}$ and esophageal pressure $Pes_{PEEP}$ in a state where the positive expiratory end pressure is non-zero, are acquired; the airway pressure $Paw_{ZEEP}$ and esophageal pressure $Pes_{ZEEP}$ in a state where the positive expiratory end pressure is zero, are acquired; and the transpulmonary pressure is added with $(Paw_{PEEP}-Paw_{ZEEP})$ and then $(Pes_{PEEP}-Pes_{ZEEP})$ is subtracted from the sum to obtain the corrected transpulmonary pressure.

In some embodiments, the transpulmonary pressure value is corrected by lung compliance and chest wall compliance in step 210. Specifically, in step 210, the lung compliance Clung and the chest wall compliance Ccw are acquired. It should be noted that there are many methods to obtain the lung compliance Clung and the chest wall compliance Ccw in step 210. For example, the chest wall compliance Ccw can be acquired through the following formula in step 210:

$$Ccw = \frac{TV}{PesI-PEEP_{es}}.$$

Wherein, TV is tidal volume, PesI is esophageal pressure at inspiratory end, $PEEP_{es}$ is esophageal pressure at expiratory end. Then the total compliance $C_{state}$ can be calculated through the following formula:

$$C_{state} = \frac{TV}{Pplat-PEEP}.$$

Wherein, TV is tidal volume, Pplat is plateau pressure, PEEP is positive airway pressure at expiratory end. When the total compliance $C_{state}$ and the chest wall compliance Ccw are calculated, the lung compliance Clung can be calculated by solving the following equation:

$$\frac{1}{Clung} + \frac{1}{Ccw} = \frac{1}{C_{state}}.$$

After acquiring the lung compliance Clung and the chest wall compliance Ccw, an error compensation value can be calculated through the following formula by a processor 50:

$$\Delta Ptrans_{erro} = Ptrans - Plung * \frac{\frac{1}{Clung}}{\frac{1}{Clung}+\frac{1}{Ccw}}.$$

Wherein, $\Delta Ptrans_{erro}$ is the error compensation value, Ptrans is the transpulmonary pressure value, Plung is the intrapulmonary pressure value.

In step 210, the error compensation value is subtracted from the transpulmonary pressure value to obtain the corrected transpulmonary pressure value.

In some embodiments, after acquiring the transdiaphragmatic pressure of the patient, the transdiaphragmatic pressure is corrected in step 200. The followings are specific description for the correction method of the transdiaphragmatic pressure in several examples.

In some embodiments, the transdiaphragmatic pressure is corrected by acquiring the esophageal pressure $Pes_{PEEP}$ and the intragastric pressure $Psto_{PEEP}$ when the positive expiratory end pressure is non-zero, and the esophageal pressure $Pes_{ZEEP}$ and the intragastric pressure $Psto_{ZEEP}$ when the positive expiratory end pressure is zero in step 210; adding the transdiaphragmatic pressure with $(Pes_{PEEP}-Pes_{ZEEP})$ and subtracting $(Psto_{PEEP}-Psto_{ZEEP})$ from the sum to obtain the corrected transdiaphragmatic pressure in step 210.

In some embodiments, step 210 may be omitted, that is, the acquired pressure of the patient is not corrected. For example, FIG. 8 above is an example in which step 210 is not included, and FIG. 9 above is an example in which step 210 is included.

In step 300, energy which acts on the respiratory system of the patient during the mechanical ventilation process, is calculated, according to the acquired pressure and the acquired gas flow rate.

In some embodiments, in step 300, the acquired pressure and gas flow rate within a preset unit time, such as 1 minute, are integrated to obtain the energy which acts on the respiratory system of the patient during the mechanical ventilation process. In some embodiments, in step 300, the acquired pressure and gas flow rate in one respiratory cycle are integrated and the integral value is multiplied by the respiratory rate to obtain the energy which acts on the respiratory system of the patient during the mechanical ventilation process. The following is a further explanation of how to calculate the energy which acts on the respiratory system of the patient during the mechanical ventilation process in combination with the pressure at different points of the respiratory system.

In some embodiments, in step 300, the energy which acts on the respiratory system of the patient during the mechanical ventilation process is calculated according to the airway pressure and the gas flow rate. For example, the energy which acts on the respiratory system of the patient during the mechanical ventilation process can be obtained by integrating the airway pressure and gas flow rate in step 300. The formula is as follows:

$$\text{Energy}_{rs} = \int_0^{\text{Tinsp}} \text{Paw} * \text{Flow dt}.$$

Wherein $\text{Energy}_{rs}$ represents energy which acts on the respiratory system of the patient during the mechanical ventilation process and is obtained by integrating the airway pressure and gas flow rate of a signal cycle, Tinsp represents inspiratory time for each respiratory cycle, Paw represents airway pressure, and Flow represents gas flow rate. Of course, the energy which is calculated in a single cycle can also be combined with respiratory rate to obtain energy of each minute, and the formula is as follows:

$$\text{Power}_{rs} = 0.098 * \text{RR} * \int_0^{\text{Tinsp}} \text{Paw} * \text{Flow dt}.$$

Wherein, unit of airway pressure Paw is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the airway pressure and gas flow rate $\text{Power}_{rs}$ is J/min. As $1cmH_2O*1L/min = 0.098J/min$, there is a coefficient of 0.098 in the above formula. Of course, the energy $\text{Energy}_{rs}$ of all cycles within 1 minute can be directly accumulated to obtain the energy per minute.

In some embodiments, when calculating the energy which acts on the respiratory system of the patient during the mechanical ventilation process according to airway pressure and gas flow rate, potential energy which is generated by the tidal volume part formed by positive expiratory end pressure can also be considered. This part of energy is generally a fixed value and never changes with the mechanical ventilation process, and it can often be omitted because of the need for additional positive expiratory end pressure release. When considering this part of potential energy, the above formula becomes:

$$\text{Energy}_{rs} = \int_0^{\text{Tinsp}} \text{Paw} * \text{Flow dt} + \frac{1}{2} * \text{PEEP}_{\text{volume}} * \text{PEEP}.$$

Then energy per minute is obtained after unit conversion combined with the respiratory rate:

$$\text{Power}_{rs} = 0.098 * \text{RR} * \left( \int_0^{\text{Tinsp}} \text{Paw} * \text{Flow dt} + \frac{1}{2} * \text{PEEP}_{\text{volume}} * \text{PEEP} \right).$$

In these two formulas, $\text{PEEP}_{\text{Volume}}$ represents tidal volume which is caused by positive expiratory end pressure and has unit L, specifically, represents volume exhaled when the positive expiratory end pressure drops to zero; PEEP represents positive expiratory end pressure.

The energy which acts on the respiratory system of the patient during the mechanical ventilation process and is calculated according to the airway pressure and gas flow rate, can represent the energy which acts on the whole respiratory system of the patient during the mechanical ventilation process, such as the total energy which acts on trachea, chest wall and lungs of the patient.

In some embodiments, in step 300, the energy which acts on the respiratory system of the patient during the mechanical ventilation process, is calculated according to values of intrapulmonary pressure and gas flow rate. It should be noted that the intrapulmonary pressure can be acquired by the pressure sensor 10 or estimated through the airway pressure, which has been described in detail above and is repeated here. In some examples, the energy which acts on the respiratory system of the patient during the mechanical ventilation process is obtained by integrating the intrapulmonary pressure and gas flow rate. The formula is as follows:

$$Energy_{lung} = \int_0^{Tinsp} Plung * Flow \, dt;$$

Wherein $Energy_{lung}$ represents energy which acts on the respiratory system of the patient during the mechanical ventilation process and is obtained by integrating the intrapulmonary pressure and gas flow rate of a signal cycle, Tinsp represents inspiratory time for each respiratory cycle, Plung represents intrapulmonary pressure, and Flow represents gas flow rate. Of course, the energy which is calculated in a single cycle can also be combined with respiratory rate to obtain energy of each minute, and the formula is as follows:

$$Power_{lung} = 0.098 * RR * \int_0^{Tinsp} Plung * Flow \, dt.$$

Wherein, unit of intrapulmonary pressure Plung is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the intrapulmonary pressure and gas flow rate $Power_{lung}$ is J/min. As $1cmH_2O* 1L/min =0.098J/min$, there is a coefficient of 0.098 in the above formula. Of course, the energy per second or hour can be calculated according to specific needs. At this time, the corresponding unit is J/s or J/h. Correspondingly, the coefficient 0.098 in the above formula corresponds to other values which are converted according to units.

In some embodiments, when calculating the energy which acts on the respiratory system of the patient during the mechanical ventilation process according to intrapulmonary pressure and gas flow rate, potential energy which is generated by the tidal volume part formed by intrapulmonary pressure at expiratory end can also be considered. This part of energy is generally a fixed value and never changes with the mechanical ventilation process, and it can often be omitted. When considering this part of potential energy, the above formula becomes:

$$Energy_{lung} = \int_0^{Tinsp} Plung * Flow \, dt + \frac{1}{2} * PlungE_{volume} * PlungE.$$

Then energy per minute is obtained after unit conversion combined with the respiratory rate:

$$Power_{lung} =$$

$$0.098 * RR * \left( \int_0^{Tinsp} Plung * Flow \, dt + \frac{1}{2} * PlungE_{volume} * PlungE \right).$$

In these two formulas, $PlungE_{Volume}$ represents tidal volume which is caused by intrapulmonary pressure at expiratory end and has unit L, specifically, represents volume exhaled when the intrapulmonary pressure at expiratory end drops to zero; PlungE represents intrapulmonary pressure at expiratory end.

The energy which acts on the respiratory system of the patient during the mechanical ventilation process and is calculated according to the intrapulmonary pressure and gas flow rate, can represent the energy of the mechanical ventilation process which acts on the chest wall and lungs of the patient.

In some embodiments, in step 300, the energy which acts on the respiratory system of the patient during the mechanical ventilation process is calculated according to values of transpulmonary pressure and gas flow rate. For example, the energy which acts on the respiratory system of the patient during the mechanical ventilation process is obtained by integrating the transpulmonary pressure and gas flow rate. The formula is as follows:

$$Energy_{tr} = \int_0^{Tinsp} Ptrans * Flow \, dt.$$

Wherein, $Energy_{tr}$ represents energy which acts on the respiratory system of the patient during the mechanical ventilation process and is obtained by integrating the transpulmonary pressure and gas flow rate of a signal cycle, Tinsp represents inspiratory time for each respiratory cycle, Ptrans represents transpulmonary pressure, and Flow represents gas flow rate. Of course, the energy which is calculated in a single cycle can also be combined with respiratory rate to obtain energy of each minute, and the formula is as follows:

$$\text{Power}_{tr} = 0.098 * RR * \int_0^{\text{Tinsp}} \text{Ptrans} * \text{Flow dt}.$$

Wherein, unit of transpulmonary pressure Ptrans is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the transpulmonary pressure and gas flow rate $\text{Power}_{tr}$ is J/min. As $1cmH_2O * 1L/min = 0.098J/min$, there is a coefficient of 0.098 in the above formula. Similarly, the unit and coefficient can also be set as required.

In some embodiments, when calculating the energy which acts on the respiratory system of the patient during the mechanical ventilation process according to transpulmonary pressure and gas flow rate, potential energy which is generated by the tidal volume part formed by transpulmonary pressure at expiratory end can also be considered. This part of energy is generally a fixed value and never changes with the mechanical ventilation process, and it can often be omitted. When considering this part of potential energy, the above formula becomes:

$$\text{Energy}_{tr} = \int_0^{\text{Tinsp}} \text{Ptrans} * \text{Flow dt} + \frac{1}{2} * \text{PtansE}_{volume} * \text{PtransE}.$$

Then energy per minute is obtained after unit conversion combined with the respiratory rate:

$$\text{Power}_{tr} = 0.098 * RR * \left( \int_0^{\text{Tinsp}} \text{Ptrans} * \text{Flow dt} + \frac{1}{2} * \text{PtansE}_{volume} * \text{PtransE} \right).$$

In these two formulas, $\text{PtansE}_{volume}$ represents tidal volume which is caused by transpulmonary pressure at expiratory end and has unit L, specifically, represents volume exhaled when the transpulmonary pressure at expiratory end drops to zero; PtransE represents transpulmonary pressure at expiratory end.

The energy which acts on the respiratory system of the patient during the mechanical ventilation process and is calculated according to the transpulmonary pressure and gas flow rate, can represent the energy of the mechanical ventilation process which acts on the lungs of the patient.

In some embodiments, in step 300, the energy which acts on the respiratory system of the patient during the mechanical ventilation process is calculated according to values of transdiaphragmatic pressure and gas flow rate. For example, the energy which acts on the respiratory system of the patient during the mechanical ventilation process is obtained by integrating the transdiaphragmatic pressure and gas flow rate. The formula is as follows:

$$\text{Energy}_{di} = \int_0^{\text{Tinsp}} \text{Pdi} * \text{Flow dt}.$$

Wherein, $\text{Energy}_{di}$ represents energy which acts on the respiratory system of the patient during the mechanical ventilation process and is obtained by integrating the transdiaphragmatic pressure and gas flow rate of a signal cycle, Tinsp represents inspiratory time for each respiratory cycle, Pdi represents transdiaphragmatic pressure, and Flow represents gas flow rate. Of course, the energy which is calculated in a single cycle can also be combined with respiratory rate to obtain energy of each minute, and the formula is as follows:

$$\text{Power}_{di} = 0.098 * RR * \int_0^{\text{Tinsp}} \text{Pdi} * \text{Flow dt}.$$

Wherein, unit of transdiaphragmatic pressure Pdi is $cmH_2O$, unit of gas flow rate Flow is L/min; unit of inspiratory time for each respiratory cycle Tinsp is s, RR represents respiratory rate, and its unit is per minute. Unit of energy which acts on the respiratory system of the patient during the mechanical ventilation process and is obtained by integrating the transdiaphragmatic pressure and gas flow rate $\text{Power}_{di}$ is J/min. As $1cmH_2O * 1L/min = 0.098J/min$, there is a coefficient of 0.098 in the above formula. Similarly, the unit and coefficient can also be set as required.

In some embodiments, when calculating the energy which acts on the respiratory system of the patient during the mechanical ventilation process according to transdiaphragmatic pressure and gas flow rate, potential energy which is generated by the tidal volume part formed by transdiaphragmatic pressure at expiratory end can also be considered. This part of energy is generally a fixed value and never changes with the mechanical ventilation process, and it can often be omitted. When considering this part of potential energy, the above formula becomes:

$$\text{Energyr}_{di} = \int_0^{\text{Tinsp}} \text{Pdi} * \text{Flow dt} + \frac{1}{2} * \text{PdiE}_{volume} * \text{PdiE}.$$

Then energy per minute is obtained after unit conversion combined with the respiratory rate:

$$\text{Power}_{di} = 0.098 * \text{RR} * \left( \int_0^{\text{Tinsp}} \text{Pdi} * \text{Flow dt} + \frac{1}{2} * \text{PdiE}_{volume} * \text{PdiE} \right).$$

In these two formulas, $\text{PdiE}_{volume}$ represents tidal volume which is caused by transdiaphragmatic pressure at expiratory end and has unit L, specifically, represents volume exhaled when the transdiaphragmatic pressure at expiratory end drops to zero; PdiE represents transdiaphragmatic pressure at expiratory end.

The energy which acts on the respiratory system of the patient during the mechanical ventilation process and is calculated according to the transdiaphragmatic pressure and gas flow rate, can represent the energy of the mechanical ventilation process which acts on the diaphragm of the patient.

In step 400, a ventilation parameter is acquired. In some embodiments, the ventilation parameter includes a ventilation control parameter and/or a respiratory system related parameter. In some embodiments, the ventilation control parameter may include one or more of tidal volume, gas flow rate, drive pressure, positive respiratory end pressure, respiratory rate, and respiratory ratio. Generally, in the mechanical ventilation process under the volume mode, the ventilation control parameters involved usually include tidal volume, gas flow rate, positive respiratory end pressure, respiratory rate and respiratory ratio. In the mechanical ventilation process under the pressure mode, the ventilation control parameters involved usually include tidal volume, drive pressure, positive respiratory end pressure, respiratory rate and respiratory ratio. In some embodiments, the respiratory system related parameter includes one or more of respiratory system compliance and respiratory system resistance.

In step 500, the degree of contribution of the ventilation parameter to the energy which acts on the respiratory system of the patient during the mechanical ventilation process, is determined. How to determine degree of contribution of the ventilation parameter to the energy which acts on the respiratory system of the patient during the mechanical ventilation process in step 500 is described below.

As stated above, the ventilation control parameter may include one or more of tidal volume, gas flow rate, drive pressure, positive respiratory end pressure, respiratory rate, and respiratory ratio. When determining that a degree of contribution of any one ventilation control parameter, other ventilation control parameters can be kept unchanged, and then the change relationship between the ventilation control parameter of which the degree of contribution is to be determined and the energy which acts on the respiratory system of the patient during the mechanical ventilation process, can be determined. For example, for each change of a fixed amount of the ventilation control parameter of which the degree of contribution is to be determined, corresponding change amount of the energy which acts on the respiratory system of the patient during the mechanical ventilation process is obtained, and then the relationship between the ventilation control parameter of which the degree of contribution is to be determined and the energy which acts on the respiratory system of the patient during the mechanical ventilation process is obtained through fitting, such as linear fitting or exponential fitting. In this way, the degree of contribution of the ventilation control parameter can be determined. Take mechanical ventilation process under the volume mode as an example. The ventilation control parameters involved in this mode usually include tidal volume, gas flow rate, positive respiratory end pressure, respiratory rate and respiratory ratio. In order to calculate the degree of contribution of tidal volume to the energy which acts on the respiratory system of the patient during the mechanical ventilation process, the gas flow rate, positive respiratory end pressure, respiratory rate and respiratory ratio can be maintained as fixed values, and then the value of tidal volume can be changed. For example, each time the tidal volume is increased by a fixed percentage X% -- for example, 2% -- and then the increased percentage Y% -- for example, Y1%, Y2%..., Yn% of the energy which acts on the respiratory system of the patient in the mechanical ventilation process is obtained. Then the relationship between X and Y is obtained by fitting. For example, assuming that K= Y/X, then the degree of contribution of tidal volume to the energy which acts on the respiratory system of the patient in the mechanical ventilation process is K. Still taking table 1 or example, the change from state 1 to state 2 is because TV in ventilation parameters changes from 500 to 1000, i.e., an increase of 100%. Correspondingly, Power increases from 5.82 to 17.4, an increase of approximately 200%. If linear fitting is performed, the rate of change or degree of contribution of TV is 2. The change from state 1 to state 3 is because the change of Rate in ventilation parameters is from 12 to 24, i.e., an increase of 100%. Correspondingly, Power increases from 5.82 to 14.46, an increase of approximately 150%. If linear fitting is performed, the rate of change or degree of contribution of Rate is 1.5. It can be understood that when the gas flow rate, positive respiratory end pressure, respiratory rate and respiratory ratio are maintained as fixed values, these fixed values affect the value of the degree of contribution of tidal volume. For example, when the gas flow rate takes two different fixed values, the degrees of contribution of tidal volume are calculated separately, and the obtained degrees of contribution of tidal volume are generally different. However, the ranking of degree of contribution of these ventilation control parameters to the energy which acts on the respiratory system of the patient during the mechanical ventilation process is generally fixed.

As discussed above, the respiratory system related parameters may include one or more of respiratory system compliance and respiratory system resistance of the patient. The degree of contribution of respiratory system compliance to the energy which acts on the respiratory system of the patient during the mechanical ventilation process can be expressed as the proportion of work which is done to overcome the respiratory system compliance in the energy which acts on the respiratory system of the patient during the mechanical ventilation process. Similarly, the degree of contribution of respiratory system resistance to the energy which acts on the respiratory system of the patient during the mechanical ventilation process can be expressed as the proportion of work which is done to overcome the respiratory system resistance in the energy which acts on the respiratory system of the patient during the mechanical ventilation process. The following is a detailed description. Please refer to FIG. 5 for the static pressure-volume curve (P-V curve) of the respiratory system, which can reflect the mechanical state of the respiratory system of the patient. The abscissa shown in the figure is the airway pressure Paw. In some other examples, the abscissa may also be the intrapulmonary pressure Plung, the transpulmonary pressure Ptrans, or the transdiaphragmatic pressure Pdi.

The abscissa of airway pressure Paw is taken for example. In the figure, A is kinetic energy part, which indicates the amount of work which is done to overcome the respiratory system resistance. In the figure, B, C and D are potential energy parts, which are related to the respiratory system compliance. Specifically, A and B are dynamically changing, and they work with the change of tidal volume and drive pressure. While C and D are static. When the drive pressure changes above the positive expiratory end pressure PEEP, the tidal volume and pressure which are related to the positive expiratory end pressure PEEP do not work, but there is still elastic potential energy, which is part of the energy which acts on the respiratory system of the patient during the mechanical ventilation process.

In one example, the potential energy part which is related to the respiratory system compliance in the figure, i.e., B, C and D, can be calculated by the following formula:

$$\text{Energy}_C = \int_0^{\text{Tinsp}} \text{Plung} * \text{Flow dt} + \frac{1}{2} * \text{PEEP}_{\text{volume}} * \text{PEEP}.$$

Wherein $\text{Energy}_C$ represents energy which is related to the respiratory system compliance in a single cycle, Tinsp represents inspiratory time for each respiratory cycle, Plung represents intrapulmonary pressure, and Flow represents gas flow rate; $\text{PEEP}_{\text{Volume}}$ represents tidal volume which is caused by positive expiratory end pressure and has unit L, specifically, represents volume exhaled when the positive expiratory end pressure drops to zero; PEEP represents positive expiratory end pressure.

In some examples, the potential energy part which is related to the respiratory system compliance may be further divided into the potential energy part which is related to respiratory system dynamic compliance and the potential energy part which is related to respiratory system static compliance. Specifically, the work which is done to overcome the respiratory system dynamic compliance in the figure, that is, the potential energy parts of B and C in the figure are:

$$\text{Energy}_{\text{Cdyn}} = \int_0^{\text{Tinsp}} \text{Plung} * \text{Flow dt}.$$

Wherein $\text{Energy}_{\text{Cdyn}}$ represents energy which is related to the respiratory system dynamic compliance in a single cycle, that is, the work which is done to overcome the respiratory system dynamic compliance, Tinsp represents inspiratory time for each respiratory cycle, Plung represents intrapulmonary pressure, and Flow represents gas flow rate.

The work which is done to overcome the respiratory system static compliance in the figure, that is, the potential energy part of D in the figure is:

$$\text{Energy}_{\text{Cstat}} = \frac{1}{2} * \text{PEEP}_{\text{volume}} * \text{PEEP}.$$

Wherein $\text{Energy}_{\text{Cstat}}$ represents energy which is related to the respiratory system static compliance in a single cycle, that is, the work which is done to overcome the respiratory system static compliance; $\text{PEEP}_{\text{Volume}}$ represents tidal volume which is caused by positive expiratory end pressure and has unit L, specifically, represents volume exhaled when the positive expiratory end pressure drops to zero; PEEP represents positive expiratory end pressure.

In one example, the work done in the figure to overcome the respiratory system resistance, that is, the kinetic energy part A, can be calculated by the following formula:

$$\mathrm{Energy}_R = \int_0^{\mathrm{Tinsp}} (\mathrm{Paw} - \mathrm{Plung}) * \mathrm{Flow \ dt}.$$

Wherein $\mathrm{Energy}_R$ represents work which is done to overcome the respiratory system resistance in a single cycle, Tinsp represents inspiratory time for each respiratory cycle, Paw represents airway pressure, Plung represents intrapulmonary pressure, and Flow represents gas flow rate.

Of course, the energy which is calculated in a single cycle can also be combined with respiratory rate to obtain energy of each minute. The energy $\mathrm{Energy}_C$, $\mathrm{Energy}_{Cdyn}$, $\mathrm{Energy}_{Cstat}$ and $\mathrm{Energy}_R$ mentioned above are described below.

The conversion of energy $\mathrm{Energy}_C$ which is related to respiratory system compliance in a single cycle is as follows:

$$\mathrm{Power}_C = 0.098 * \mathrm{RR} * \left( \int_0^{\mathrm{Tinsp}} \mathrm{Plung} * \mathrm{Flow \ dt} + \frac{1}{2} * \mathrm{PEEP}_{volume} * \mathrm{PEEP} \right).$$

The conversion of energy $\mathrm{Energy}_{Cdyn}$ which is related to respiratory system dynamic compliance in a single cycle is as follows:

$$\mathrm{Power}_{Cdyn} = 0.098 * \mathrm{RR} * \left( \int_0^{\mathrm{Tinsp}} \mathrm{Plung} * \mathrm{Flow \ dt} \right).$$

The conversion of energy $\mathrm{Energy}_{Cstat}$ which is related to respiratory system static compliance in a single cycle is as follows:

$$\mathrm{Power}_{Cstat} = 0.098 * \mathrm{RR} * \left( \frac{1}{2} * \mathrm{PEEP}_{volume} * \mathrm{PEEP} \right).$$

The conversion of work $\mathrm{Energy}_R$ which is done to overcome the respiratory system resistance in a single cycle is as follows:

$$\mathrm{Power}_R = 0.098 * \mathrm{RR} * \left( \int_0^{\mathrm{Tinsp}} (\mathrm{Paw} - \mathrm{Plung}) * \mathrm{Flow \ dt} \right).$$

In the above formula, units of airway pressure Paw and intrapulmonary pressure Plung are $cmH_2O$, unit of gas flow rate Flow is L/min; unit of inspiratory time for each respiratory cycle Tinsp is s, RR represents respiratory rate, and its unit is per minute. Units of energy $\mathrm{Power}_C$ which is related to respiratory system compliance for each minute, energy $\mathrm{Power}_{Cdyn}$ which is related to respiratory system dynamic compliance for each minute, energy $\mathrm{Power}_{Cstat}$ which is related to respiratory system static compliance for each minute and work $\mathrm{Power}_R$ which is done to overcome the respiratory system resistance for each minute are J/min. As $1cmH_2O*1$L/min =0.098J/min, there is a coefficient of 0.098 in the above formula. As $1cmH_2O*1$L/min =0.098J/min, there is a coefficient of 0.098 in the above formula.

Of course, the energy $\mathrm{Energy}_C$ of all cycles within 1 minute can be directly accumulated to obtain the energy per minute $\mathrm{Power}_C$; the energy $\mathrm{Energy}_{Cdyn}$ of all cycles within 1 minute can be directly accumulated to obtain the energy per minute $\mathrm{Power}_{Cdyn}$; the energy $\mathrm{Energy}_{Cstat}$ of all cycles within 1 minute can be directly accumulated to obtain the energy per minute $\mathrm{Power}_{Cstat}$; the work $\mathrm{Energy}_R$ of all cycles within 1 minute can be directly accumulated to obtain the work per minute $\mathrm{Power}_R$.

The above is to calculate the degree of contribution of the respiratory system related parameters to the energy which acts on the respiratory system of the patient during the mechanical ventilation process by the integral method. In some embodiments, the degree of contribution of the respiratory system related parameters to the energy which acts on the respiratory system of the patient during the mechanical ventilation process can also be calculated by the formula method.

Similar to FIG.5, FIG.6 is also the static pressure-volume curve (P-V curve) of the respiratory system. The abscissa of airway pressure Paw in FIG.6 is taken for example. The energy which acts on the respiratory system of the patient during the mechanical ventilation process is as follows:

$$\text{Energy}_{rs} = \left[TV * \left(\frac{500*TV}{Crs} + RR * \frac{(1+I:E)*TV*Raw}{60*I:E}\right) + TV * PEEP\right].$$

Wherein Energy$_{rs}$ represents energy which acts on the respiratory system of the patient during the mechanical ventilation process of a signal cycle, TV represents tidal volume, Crs represents respiratory system compliance, RR represents respiratory rate, I: E represents a ratio of inspiratory time and expiratory time, that is the respiratory ratio; Raw represents respiratory system resistance, PEEP represents positive expiratory end pressure. The energy which is calculated in a single cycle can also be combined with respiratory rate to obtain energy of each minute, and the formula is as follows:

$$\text{Power}_{rs} = 0.098 * RR * \left[TV * \left(\frac{500*TV}{Crs} + RR * \frac{(1+I:E)*TV*Raw}{60*I:E}\right) + TV * PEEP\right].$$

Wherein unit of energy for each minute Power$_{rs}$ is J/min; unit of respiratory rate RR is per minute; unit of tidal volume TV is L; unit of respiratory system compliance Crs is ml/cmH$_2$O; unit of respiratory system resistance Raw is cmH$_2$O/L/s; unit of positive expiratory end pressure PEEP is cmH$_2$O.

In general, when calculating the energy which acts on the respiratory system of the patient during the mechanical ventilation process, potential energy which is generated by the tidal volume part formed by positive expiratory end pressure can also be considered. This part of energy is generally a fixed value and never changes with the mechanical ventilation process, and it can often be omitted because of the need for additional positive expiratory end pressure release. When considering this part of potential energy, the above formula becomes:

$$\text{Energy}_{rs} = \left[TV * \left(\frac{500*TV}{Crs} + RR * \frac{(1+I:E)*TV*Raw}{60*I:E}\right) + TV * PEEP + \frac{1}{2} * PEEP_{volume} * PEEP\right].$$

Then energy per minute is obtained after unit conversion combined with the respiratory rate:

$$\text{Power}_{rs} = 0.098 * RR * \left[TV * \left(\frac{500*TV}{Crs} + RR * \frac{(1+I:E)*TV*Raw}{60*I:E}\right) + TV * PEEP + \frac{1}{2} * PEEP_{volume} * PEEP\right].$$

In these two formulas, PEEP$_{Volume}$ represents tidal volume which is caused by positive expiratory end pressure and has unit L, specifically, represents volume exhaled when the positive expiratory end pressure drops to zero.

The degree of contribution of respiratory system related parameter is calculated below. Taking energy of a single cycle which acts on the respiratory system of the patient during the mechanical ventilation process as

$$\text{Energy}_{rs} = \left[TV * \left(\frac{500*TV}{Crs} + RR * \frac{(1+I:E)*TV*Raw}{60*I:E}\right) + TV * PEEP\right]$$, and energy of each minute as

$$\text{Power}_{rs} = 0.098 * RR * \left[TV * \left(\frac{500*TV}{Crs} + RR * \frac{(1+I:E)*TV*Raw}{60*I:E}\right) + TV * PEEP\right]$$ for example, it can be seen from the respiratory mechanics equation that the following equation holds:

$$\left(\frac{TV*1000}{Crs}\right) = P_{plat} - PEEP;$$

$$RR * \frac{(1+I:E)TV}{60*I:E} = \frac{TV}{Tinsp} = Flow;$$

$$\text{Flow} * \text{Raw} = \left(\text{Ppeak} - P_{\text{plat}}\right).$$

Wherein, Pplat is plateau pressure, Tinsp represents inspiratory time for each respiratory cycle, Ppeak represents peak pressure shown in the figure, Flow represents gas flow rate. Therefore, it can be deduced that:

$$\text{Energy}_{\text{rs}} = \left[\frac{\text{TV}*\left(P_{\text{plat}}-\text{PEEP}\right)}{2} + \text{TV} * \left(\text{Ppeak} - P_{\text{plat}}\right) + TV * PEEP\right];$$

$$\text{Pawer}_{\text{rs}} = 0.098 * \text{RR} * \left[\frac{\text{TV}*\left(P_{\text{plat}}-\text{PEEP}\right)}{2} + \text{TV} * \left(\text{Ppeak} - P_{\text{plat}}\right) + TV * PEEP\right].$$

Therefore, the work which is done to overcome the respiratory system resistance is:

$$\text{Energy}_{\text{R}} = \text{TV} * \left(\text{Ppeak} - \text{Pplat}\right) = TV * RR * \frac{(1+\text{I:E})\text{TV}*\text{Raw}}{60*\text{I:E}}.$$

The work which is done to overcome the respiratory system dynamic compliance is:

$$\text{Energy}_{\text{Cdyn}} = \text{TV} * PEEP.$$

The work which is done to overcome the respiratory system static compliance is:

$$\text{Energy}_{\text{Csta}} = \frac{\text{TV}*\left(P_{\text{plat}}-\text{PEEP}\right)}{2} = \frac{500*\text{TV}*\text{TV}}{\text{Crs}}.$$

The work which is done to overcome the respiratory system compliance is equal to the work which is done to overcome the respiratory system dynamic compliance plus the work which is done to overcome the respiratory system static compliance.

Further, the ratio of the work which is done to overcome the respiratory system compliance to the energy which acts on the respiratory system of the patient during the mechanical ventilation process can also be calculated to represent the degree of contribution of the respiratory system compliance. Similarly, the ratio of the work which is done to overcome the respiratory system resistance to the energy which acts on the respiratory system of the patient during the mechanical ventilation process is calculated to represent the degree of contribution of the respiratory system resistance.

The above is some description of the degree of contribution of ventilation parameters, including ventilation control parameters and/or respiratory system related parameters, to the energy which acts on the respiratory system of the patient during the mechanical ventilation process. After determining the degree of contribution of ventilation parameters to the energy which acts on the respiratory system of the patient during the mechanical ventilation process, there are many ways to use the degree of contribution, which are described below.

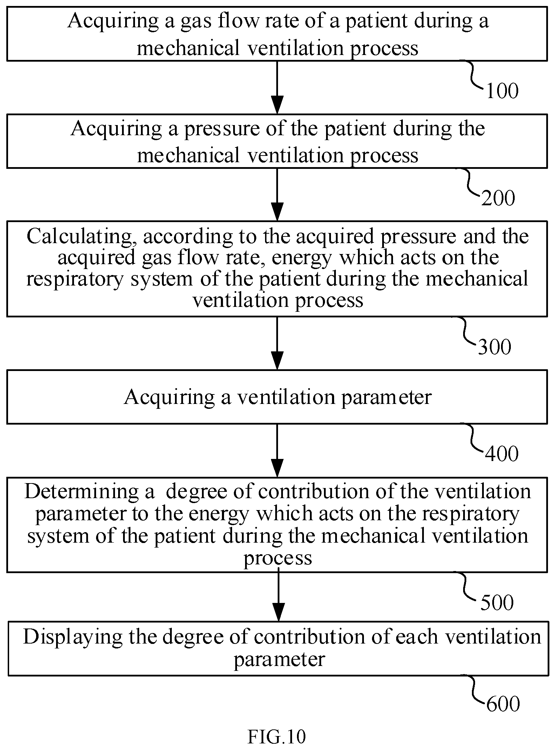

Referring FIG. 10, in some embodiments, the method for monitoring ventilation of a patient, further include step 600, in which the degree of contribution of each ventilation parameter is displayed in one or more ways such as word, number, character, table, diagram or icon. For example, in step 600, name of each ventilation parameter and value of its corresponding degree of contribution are displayed. In some specific embodiments, the name of each ventilation parameter and its corresponding degree of contribution value are displayed in the order of ranking the degree of contribution from large to small or from small to large, so that medical personnel can easily understand the degree of contribution of each parameter to the energy which acts on the respiratory system of the patient during the mechanical ventilation process. For example, in an example of mechanical ventilation process under the volume mode, in step 600, the degree of contribution of ventilation control parameters involved in the mode, such as tidal volume, gas flow rate, positive respiratory end pressure, respiratory rate, and respiratory ratio, are displayed. For another example, in an example of mechanical ventilation process under a pressure mode, the degree of contribution of ventilation control parameters involved in the mode, such as tidal volume, drive pressure, positive respiratory end pressure, respiratory rate and respiratory ratio, are displayed. Of course, in some examples, whether under the volume mode or the pressure mode, in step 600, the degree of contribution of respiratory system related parameters such as the respiratory system compliance and respiratory system resistance of the patient, are displayed. Therefore, in some examples, in step 600, the name of each ventilation control parameter and the value of its corresponding degree of contribution, are displayed. Of course, in some examples, in step 600, the name of each ventilation control parameter and the ranking of its corresponding degree of contribution, are displayed. In some other examples, in step 600, the degree of contribution of each ventilation control parameter, can be graphically displayed. In some other examples, in step 600, the name of each respiratory system related parameter and the value of its corresponding degree of contribution, can be displayed. In some other examples, in step 600, proportion of degrees of contribution of each respiratory system related parameter can be displayed. In some other examples, in step 600, the degree of contribution of each respiratory system related parameter, can be graphically displayed. Accordingly, the value of the degree of contribution can be reflected in various ways, such as monitored value, percentage or rate of change, and so on. Taking the respiratory system compliance as an example, displaying the value of its degree of contribution can be displaying the actual monitored value, percentage or rate of change of the work which is done to overcome the respiratory system compliance in the energy which acts on the respiratory system of the patient during the mechanical ventilation process. Similarly, displaying the value of the degree of contribution of the respiratory system resistance, may also be displaying the actual monitored value, percentage or rate of change of the work which is done to overcome the respiratory system resistance in the energy which acts on the respiratory system of the patient during the mechanical ventilation process. In some embodiments, in addition to the above-mentioned display of the name of the ventilation parameter and the corresponding value of the degree of contribution, the degree of contribution of each ventilation parameter can also be displayed graphically, such as through a bar chart, a pie chart or a table. The above table 2, FIG. 7 (a) and FIG. 7 (b) are examples. The other manners may refer to the above description and are not repeated herein.

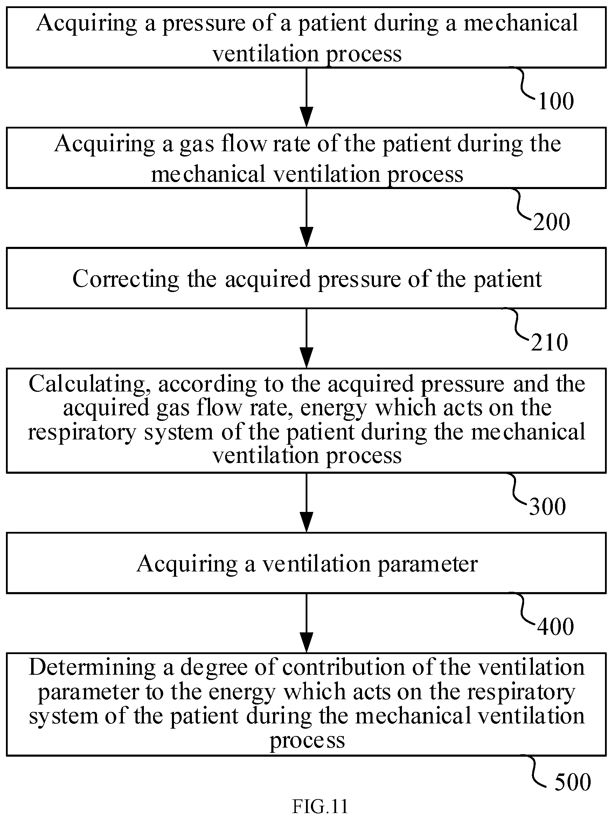

Referring FIG. 11, in some embodiments, the method for monitoring ventilation of a patient, further include step 700, in which the mechanical ventilation process is guided according to the degree of contribution of ventilation parameter. The following is a detailed description for how to guide the mechanical ventilation process according to the degree of contribution of ventilation parameter.

In some embodiments, when determining that the energy which acts on the respiratory system of the patient during the mechanical ventilation process, exceeds a first threshold, indicate to decrease the ventilation parameter according to a ranking of the degree of contribution or control to decrease the ventilation parameter according to a ranking of the degree of contribution in step 700. Specifically, it is possible to indicate to preferentially decrease the ventilation parameter with the largest degree of contribution among the ventilation control parameters, or control to preferentially decrease the ventilation parameter with the largest degree of contribution among the ventilation control parameters. When determining that the energy which acts on the respiratory system of the patient during the mechanical ventilation process, exceeds a relatively safe threshold, the ventilation parameter with the largest degree of contribution among the ventilation control parameters can preferentially be manually or automatically decreased at this time, so that the energy which acts on the respiratory system of the patient during the mechanical ventilation process, can be quickly decreased to a safe range. In some embodiments, when determining that the energy which acts on the respiratory system of the patient during the mechanical ventilation process, is lower than a second threshold, indicate to increase the ventilation parameter according to a ranking of the degree of contribution or control to increase the ventilation parameter according to a ranking of the degree of contribution in step 700. Specifically, it is possible to indicate to preferentially increase the ventilation parameter with the smallest degree of contribution among the ventilation control parameters, or control to preferentially increase the ventilation parameter with the smallest degree of contribution among the ventilation control parameters. When determining that the energy which acts on the respiratory system of the patient during the mechanical ventilation process, is lower than a relatively safe threshold, the ventilation parameter with the smallest degree of contribution among the ventilation control parameters can preferentially be manually or automatically increased, so that the energy which acts on the respiratory system of the patient during the mechanical ventilation process, can enter the safe range more smoothly and safely and would not exceed the standard amount and cause additional problems.

In some embodiments, in step 700, the energy which acts on the respiratory system of the patient during the mechanical ventilation process, is estimated according to a setting command of the ventilation control parameter and then outputted the energy after setting the ventilation control parameter. Specifically, the estimated energy which acts on the respiratory system of the patient during the mechanical ventilation may be displayed for medical personnel to view and make decisions. In some embodiments, in step 700, whether to send an alarm is determined according to the estimated energy which acts on the respiratory system of the patient during the mechanical ventilation process. For example, if the estimated energy which acts on the respiratory system of the patient during the mechanical ventilation process exceeds the first threshold or is lower than the second threshold, an alarm is send in step 700.

Some studies show that excessive energy which acts on the respiratory system of the patient during the mechanical ventilation process, has a significant clinical correlation with lung injury. Specifically, some clinical studies show that when the energy which acts on the whole respiratory system of the patient during the mechanical ventilation process is larger than 25J /min, or the energy which acts on the lungs of the patient during the mechanical ventilation process is larger than 12J/min or 13J/min and so on, it significantly cause lung injury. Some clinical studies show that when the energy which acts on the whole respiratory system of the patient during the mechanical ventilation process is larger than 17J /min, it significantly increase the mortality of patients. Therefore, the first threshold and the second threshold can be set according to the clinical data and the specific situation of the patient.

In some embodiments, in step 700, whether to send an alarm is determined according to the proportion of the degree of contribution of each respiratory system related parameter. For example, in step 700, different alarms are sent according to the proportion of the degree of contribution of the respiratory system resistance, that is, when the work which is done to overcome the respiratory system resistance accounts for the energy which acts on the respiratory system of the patient during the mechanical ventilation process in different ranges, different alarms are sent. Similarly, in step 700, different alarms are sent according to the proportion of the degree of contribution of the respiratory system compliance, that is, when the work which is done to overcome the respiratory system compliance accounts for the energy which acts on the respiratory system of the patient during the mechanical ventilation process in different ranges, different alarms are sent. The corresponding relationship between the specific proportion range and the specific alarm can be determined and preset by those skilled in the art according to the clinical data. For example, when the proportion of the degree of contribution of the respiratory system compliance is between 80% and 85%, no alarm is sent, and it is currently normal. When the proportion of the degree of contribution of the respiratory system compliance is between 70% and 75%, an alarm that the patient currently presents acute respiratory distress syndrome (ARDS) is sent. When the proportion of the degree of contribution of the respiratory system compliance is between 40% and 50%, an alarm that the patient is currently suffering from chronic obstructive pulmonary disease, is sent. Alternatively, when the degree of contribution of the respiratory system compliance is not in the range from 80% to 85%, an alarm is sent. Otherwise, no alarm is sent.

Various exemplary embodiments are described herein. However, those skilled in the art recognizes that changes and modifications may be made to the exemplary embodiments without departing from the scope of this disclosure. For example, the various operation steps and the components configured to perform the operation steps can be implemented in different ways according to the specific application or considering any figure of cost functions associated with the operation of the system (for example, one or more steps can be deleted, modified or combined into other steps).

In addition, as understood by those skilled in the art, the principles herein can be reflected in a computer program product on a computer-readable storage medium which is pre-loaded with computer-readable program code. Any tangible, non-transitory computer-readable storage medium can be used, including magnetic storage devices (hard disk, floppy disk, etc.), optical storage devices (CD-ROM, DVD, Blu ray disk, etc.), flash memory and/or the likes. Computer program instructions can be loaded onto a general-purpose computer, a special-purpose computer, or other programmable data processing device to form a machine, so that these instructions executed on a computer or other programmable data processing device can generate a device to realize a specified function. These computer program instructions may also be stored in a computer-readable memory, which may instruct a computer or other programmable data processing device to operate in a specific manner, so that the instructions stored in the computer-readable memory can form a manufactured article, including an implementation device for realizing a specified function. Computer program instructions can also be loaded on a computer or other programmable data processing device, so that a series of operation steps are performed on the computer or other programmable device to generate a computer implemented process, so that the instructions executed on the computer or other programmable device can provide steps for realizing the specified functions.

Those skilled in the art recognize that many changes can be made to the details of the above embodiments without departing from the basic principles of this disclosure. Therefore, the scope of this disclosure shall be determined according to the following attached claims.

**Claims**

1.    A method for monitoring ventilation of a patient, comprising:

acquiring a pressure of the patient during a mechanical ventilation process;
acquiring a gas flow rate of the patient during the mechanical ventilation process;
calculating, according to the acquired pressure and the acquired gas flow rate, energy which acts on the respiratory system of the patient during the mechanical ventilation process; and
acquiring a ventilation parameter;
**characterized in that**, the method for monitoring ventilation of a patient further comprises:

determining a degree of contribution of the ventilation parameter to the energy which acts on the respiratory system of the patient during the mechanical ventilation process;
wherein the pressure reflects a pressure which acts on different sites of a respiratory system of the patient during the mechanical ventilation process.

2.    The method for monitoring ventilation of a patient according to claim 1, **characterized in that**, the ventilation parameter comprises a ventilation control parameter and/or a respiratory system related parameter; wherein the ventilation control parameter comprises one or more of tidal volume, gas flow rate, drive pressure, positive respiratory end pressure, respiratory rate and respiratory ratio, and the respiratory system related parameter comprises one or more of respiratory system compliance and respiratory system resistance.

3. The method for monitoring ventilation of a patient according to claim 2, **characterized in that**, further comprising:

displaying the degree of contribution of each ventilation parameter;
preferably, the degree of contribution of each ventilation parameter is displayed in one or more manners of word, number, character, table, diagram or icon.

4. The method for monitoring ventilation of a patient according to any one of claims 1-3, **characterized in that**, further comprising:
guiding the mechanical ventilation process according to the degree of contribution.

5. The method for monitoring ventilation of a patient according to claim 4, **characterized in that**, guiding the mechanical ventilation process according to the degree of contribution, comprises:
indicating to decrease the ventilation parameter according to a ranking of the degree of contribution or controlling to decrease the ventilation parameter according to a ranking of the degree of contribution, when determining that the energy which acts on the respiratory system of the patient during the mechanical ventilation process, exceeds a first threshold.

6. The method for monitoring ventilation of a patient according to claim 4, **characterized in that**, guiding the mechanical ventilation process according to the degree of contribution, comprises:
indicating to increase the ventilation parameter according to a ranking of the degree of contribution or controlling to increase the ventilation parameter according to a ranking of the degree of contribution, when determining that the energy which acts on the respiratory system of the patient during the mechanical ventilation process, is lower than a second threshold.

7. The method for monitoring ventilation of a patient according to claim 4, **characterized in that**, guiding the mechanical ventilation process according to the degree of contribution, comprises:
estimating and outputting the energy which acts on the respiratory system of the patient, during the mechanical ventilation process which is set according to a setting command of a ventilation control parameter.

8. The method for monitoring ventilation of a patient according to claim 7, **characterized in that**, guiding the mechanical ventilation process according to the degree of contribution, further comprises:
determining whether to send an alarm according to the estimated energy which acts on the respiratory system of the patient, during the mechanical ventilation process.

9. The method for monitoring ventilation of a patient according to claim 4, **characterized in that**, guiding the mechanical ventilation process according to the degree of contribution, comprises:
determining whether to send an alarm according to the degree of contribution of each respiratory system related parameter.

10. An apparatus for monitoring ventilation of a patient, comprising:

a pressure sensor, which is configured to acquire a pressure of a patient during a mechanical ventilation process;
a flow sensor, which is configured to acquire a gas flow rate of the patient during the mechanical ventilation process; and
a processor, which is configured to acquire the pressure of the patient during the mechanical ventilation process and the gas flow rate of the patient during the mechanical ventilation process, and to calculate, according to the acquired pressure and the acquired gas flow rate, energy which acts on the respiratory system of the patient during the mechanical ventilation process;
wherein the processor is further configured to acquire a ventilation parameter;
**characterized in that**, the processor is further configured to determine a degree of contribution of the ventilation parameter to the energy which acts on the respiratory system of the patient during the mechanical ventilation process;
wherein the pressure reflects a pressure which acts on different sites of a respiratory system of the patient during the mechanical ventilation process.

11. The apparatus for monitoring ventilation of a patient according to claim 10, **characterized in that**, the ventilation parameter comprises a ventilation control parameter and/or a respiratory system related parameter; wherein the ventilation control parameter comprises one or more of tidal volume, a gas flow rate, drive pressure, positive respiratory end pressure, respiratory rate and respiratory ratio, and the respiratory system related parameter comprises one or more of respiratory system compliance and respiratory system resistance.

12. The apparatus for monitoring ventilation of a patient according to claim 10 or claim 11, **characterized in that**, the processor is further configured to guide the mechanical ventilation process according to the degree of contribution.

13. The apparatus for monitoring ventilation of a patient according to claim 12, **characterized in that**, in order to guide the mechanical ventilation process according to the degree of contribution, the processor is further configured to:

indicate to preferentially decrease the ventilation parameter with a largest degree of contribution among ventilation control parameters, or control to preferentially decrease the ventilation parameter with a largest degree of contribution among ventilation control parameters, when determining that the energy which acts on the respiratory system of the patient during the mechanical ventilation process, exceeds a first threshold; and/or indicate to preferentially increase the ventilation parameter with a smallest degree of contribution among ventilation control parameters, or control to preferentially increase the ventilation parameter with a smallest degree of contribution among ventilation control parameters, when determining that the energy which acts on the respiratory system of the patient during the mechanical ventilation process, is lower than a second threshold.

14. The apparatus for monitoring ventilation of a patient according to claim 12, **characterized in that**, in order to guide the mechanical ventilation process according to the degree of contribution, the processor is further configured to:

estimate and output the energy which acts on the respiratory system of the patient, during the mechanical ventilation process which is set according to a setting command of a ventilation control parameter, and determine whether to send an alarm according to the estimated energy which acts on the respiratory system of the patient, during the mechanical ventilation process; or
in order to guide the mechanical ventilation process according to the degree of contribution, the processor is further configured to:
determine whether to send an alarm according to the degree of contribution of each respiratory system related parameter.

15. The apparatus for monitoring ventilation of a patient according to claim 10, **characterized in that**, the apparatus for monitoring ventilation of a patient is a patient monitor, a patient monitoring module or a medical ventilation apparatus; and/or
the apparatus for monitoring ventilation of a patient further comprises a display, which is configured to display the degree of contribution of each ventilation parameter.

**Patentansprüche**

1. Ein Verfahren zur Überwachung der Beatmung eines Patienten, umfassend:

das Erfassen eines Drucks des Patienten während eines mechanischen Beatmungsvorgangs;
das Erfassen einer Gasdurchflussrate des Patienten während des mechanischen Beatmungsprozesses;
das Berechnen der Energie, die während des mechanischen Beatmungsvorgangs auf das Atmungssystem des Patienten wirkt, anhand des erfassten Drucks und der erfassten Gasflussrate; und
das Erfassen eines Beatmungsparameters;
**dadurch gekennzeichnet, dass** das Verfahren zur Überwachung der Beatmung eines Patienten ferner Folgendes umfasst:

das Bestimmen eines Grad des Beitrags des Beatmungsparameters zu der Energie, die während des mechanischen Beatmungsprozesses auf das Atmungssystem des Patienten wirkt;
wobei der Druck einen Druck wiederspiegelt, der während des mechanischen Beatmungsprozesses auf verschiedene Stellen des Atmungssystems des Patienten wirkt.

2. Das Verfahren zur Überwachung der Beatmung eines Patienten nach Anspruch 1, **dadurch gekennzeichnet, dass** der Beatmungsparameter einen Beatmungssteuerungsparameter und/oder einen Parameter umfasst, der sich auf das Atmungssystem bezieht, wobei der Beatmungssteuerungsparameter eines oder mehrere der folgenden Elemente umfasst: Atemzugvolumen, Gasdurchflussrate, Antriebsdruck, positiver Atemenddruck, Atemfrequenz und Atemverhältnis, und der Parameter, der sich auf das Atmungssystem bezieht, eines oder mehrere der folgenden Elemente umfasst: Compliance des Atmungssystems und Widerstand des Atmungssystems.

3. Das Verfahren zur Überwachung der Beatmung eines Patienten nach Anspruch 2, **dadurch gekennzeichnet, dass** es ferner Folgendes umfasst:

Anzeigen des Grads des Beitrags jedes Beatmungsparameters;
vorzugsweise wird der Grad des Beitrags jedes Beatmungsparameters auf eine oder mehrere Arten angezeigt, beispielsweise durch Wörter, Zahlen, Zeichen, Tabellen oder Diagrammsymbole.

4. Das Verfahren zur Überwachung der Beatmung eines Patienten nach einem der Ansprüche 1 bis 3, **dadurch gekennzeichnet, dass** es ferner Folgendes umfasst:
die Steuerung des mechanischen Beatmungsprozesses entsprechend dem Grad des Beitrags.

5. Das Verfahren zur Überwachung der Beatmung eines Patienten nach Anspruch 4, **dadurch gekennzeichnet, dass** das Steuern des mechanischen Beatmungsprozesses entsprechend dem Grad des Beitrags Folgendes umfasst:
das Anzeigen einer Verringerung des Beatmungsparameters entsprechend einer Rangfolge des Grads des Beitrags oder das Steuern einer Verringerung des Beatmungsparameters entsprechend einer Rangfolge des Grads des Beitrags, wenn festgestellt wird, dass die Energie, die während des mechanischen Beatmungsprozesses auf das Atmungssystem des Patienten wirkt, einen ersten Schwellenwert überschreitet.

6. Das Verfahren zur Überwachung der Beatmung eines Patienten nach Anspruch 4, **dadurch gekennzeichnet, dass** das Steuern des mechanischen Beatmungsprozesses entsprechend dem Grad des Beitrags Folgendes umfasst:
das Anzeigen einer Erhöhung des Beatmungsparameters entsprechend einer Rangfolge des Grads des Beitrags oder das Steuern einer Erhöhung des Beatmungsparameters entsprechend einer Rangfolge des Grads des Beitrags, wenn festgestellt wird, dass die Energie, die während des mechanischen Beatmungsprozesses auf das Atmungssystem des Patienten wirkt, unter einem zweiten Schwellenwert liegt.

7. Das Verfahren zur Überwachung der Beatmung eines Patienten nach Anspruch 4, **dadurch gekennzeichnet, dass** das Steuern des mechanischen Beatmungsprozesses entsprechend dem Grad des Beitrags Folgendes umfasst:
das Schätzen und Ausgeben der Energie, die während des mechanischen Beatmungsprozesses, der gemäß einem Einstellbefehl eines Beatmungssteuerungsparameters eingestellt ist, auf das Atmungssystem des Patienten wirkt.

8. Das Verfahren zur Überwachung der Beatmung eines Patienten nach Anspruch 7, **dadurch gekennzeichnet, dass** das Steuern des mechanischen Beatmungsprozesses entsprechend dem Grad des Beitrags ferner Folgendes umfasst:
das Bestimmen, ob ein Alarm entsprechend der geschätzten Energie, die während des mechanischen Beatmungsprozesses auf das Atmungssystem des Patienten wirkt, ausgegeben werden soll.

9. Das Verfahren zur Überwachung der Beatmung eines Patienten nach Anspruch 4, **dadurch gekennzeichnet, dass** das Steuern des mechanischen Beatmungsprozesses entsprechend dem Grad des Beitrags Folgendes umfasst:
das Bestimmen, ob ein Alarm entsprechend dem Grad des Beitrags jedes Parameters, der sich auf das Atmungssystem bezieht, ausgegeben werden soll.

10. Eine Vorrichtung zur Überwachung der Beatmung eines Patienten, umfassend:

einen Drucksensor, der so ausgelegt ist, dass er den Druck eines Patienten während eines mechanischen Beatmungsprozesses erfasst;
einen Durchflusssensor, der so ausgelegt ist, dass er eine Gasdurchflussrate des Patienten während des mechanischen Beatmungsprozesses erfasst; und
einen Prozessor, der so ausgelegt ist, dass er den Druck des Patienten während des mechanischen Beatmungsprozesses und die Gasflussrate des Patienten während des mechanischen Beatmungsprozesses erfasst und anhand des erfassten Drucks und der erfassten Gasflussrate die Energie berechnet, die während des mechanischen Beatmungsprozesses auf das Atmungssystem des Patienten wirkt;
wobei der Prozessor ferner so ausgelegt ist, dass er einen Beatmungsparameter erfasst; **dadurch gekenn-**

zeichnet, dass der Prozessor ferner so ausgelegt ist, dass er einen Beitrag des Beatmungsparameters zu der Energie bestimmt, die während des mechanischen Beatmungsprozesses auf das Atmungssystem des Patienten wirkt;

wobei der Druck einen Druck wiederspiegelt, der während des mechanischen Beatmungsprozesses auf verschiedene Stellen eines Atmungssystems des Patienten wirkt.

11. Die Vorrichtung zur Überwachung der Beatmung eines Patienten nach Anspruch 10, **dadurch gekennzeichnet, dass** der Beatmungsparameter einen Beatmungssteuerungsparameter und/oder einen Parameter umfasst, der sich auf das Atmungssystem bezieht; wobei der Beatmungssteuerungsparameter eines oder mehrere der folgenden Elemente umfasst: Atemzugvolumen, Gasdurchflussrate, Antriebsdruck, positiver Atemenddruck, Atemfrequenz und Atemverhältnis, und der Parameter, der sich auf das Atmungssystem bezieht, eines oder mehrere der folgenden Elemente umfasst: Compliance des Atmungssystems und Widerstand des Atmungssystems.

12. Die Vorrichtung zur Überwachung der Beatmung eines Patienten nach Anspruch 10 oder 11, **dadurch gekennzeichnet, dass** der Prozessor ferner so ausgelegt ist, dass er den mechanischen Beatmungsprozess entsprechend dem Grad des Beitrags steuert.

13. Die Vorrichtung zur Überwachung der Beatmung eines Patienten nach Anspruch 12, **dadurch gekennzeichnet, dass** der Prozessor, um den mechanischen Beatmungsprozess entsprechend dem Grad des Beitrags zu steuern, ferner so ausgelegt ist, dass er:

den Beatmungsparameter mit dem größten Beitrag unter den Beatmungssteuerungsparametern vorrangig verringert oder den Beatmungsparameter mit dem größten Beitrag unter den Beatmungssteuerungsparametern vorrangig verringert, wenn festgestellt wird, dass die Energie, die während des mechanischen Beatmungsprozesses auf das Atmungssystem des Patienten wirkt, einen ersten Schwellenwert überschreitet; und/oder den Beatmungsparameter mit dem geringsten Beitrag unter den Beatmungssteuerungsparametern vorrangig erhöht oder den Beatmungsparameter mit dem geringsten Beitrag unter den Beatmungssteuerungsparametern vorrangig erhöht, wenn festgestellt wird, dass die Energie, die während des mechanischen Beatmungsprozesses auf das Atmungssystem des Patienten wirkt, unter einem zweiten Schwellenwert liegt.

14. Die Vorrichtung zur Überwachung der Beatmung eines Patienten nach Anspruch 12, **dadurch gekennzeichnet, dass** der Prozessor, um den mechanischen Beatmungsprozess entsprechend dem Beitrag zu steuern, ferner so ausgelegt ist, dass

er die Energie, die während des mechanischen Beatmungsprozesses, der gemäß einem Einstellbefehl eines Beatmungssteuerungsparameters eingestellt ist, auf das Atmungssystem des Patienten wirkt, schätzt und ausgibt und festlegt, ob ein Alarm entsprechend der geschätzten Energie, die während des mechanischen Beatmungsprozesses auf das Atmungssystem des Patienten wirkt, ausgegeben werden soll; oder um den Prozess der mechanischen Beatmung entsprechend dem Grad des Beitrags zu steuern, der Prozessor ferner so ausgelegt ist, dass er: bestimmt, ob entsprechend dem Grad des Beitrags jedes Parameters, der sich auf das Atmungssystem bezieht, ein Alarm ausgegeben werden soll.

15. Die Vorrichtung zur Überwachung der Beatmung eines Patienten nach Anspruch 10, **dadurch gekennzeichnet, dass** die Vorrichtung zur Überwachung der Beatmung eines Patienten ein Patientenmonitor, ein Patientenüberwachungsmodul oder ein medizinisches Beatmungsgerät ist, und/oder die Vorrichtung zur Überwachung der Beatmung eines Patienten ferner eine Anzeige umfasst, die so ausgelegt ist, dass sie den Grad des Beitrags jedes Beatmungsparameters anzeigt.

**Revendications**

1. Procédé de surveillance de la ventilation d'un patient, comprenant les étapes consistant à :

acquérir une pression du patient durant un processus de ventilation mécanique ;
acquérir un débit gazeux du patient durant le processus de ventilation mécanique ;

calculer, en fonction de la pression acquise et du débit gazeux acquis, une énergie qui agit sur le système respiratoire du patient durant le processus de ventilation mécanique ; et

acquérir un paramètre de ventilation ;

**caractérisé en ce que** le procédé de surveillance de la ventilation d'un patient comprend en outre :

le fait de déterminer un degré de contribution du paramètre de ventilation à l'énergie qui agit sur le système respiratoire du patient durant le processus de ventilation mécanique ;

où la pression reflète une pression qui agit sur différents sites d'un système respiratoire du patient durant le processus de ventilation mécanique.

2. Procédé de surveillance de la ventilation d'un patient selon la revendication 1, **caractérisé en ce que** le paramètre de ventilation comprend un paramètre de contrôle de ventilation et/ou un paramètre lié au système respiratoire ; où le paramètre de contrôle de ventilation comprend un ou plusieurs éléments parmi un volume courant, un débit gazeux, une pression d'entraînement, une pression positive en fin de respiration, une fréquence respiratoire et un ratio respiratoire, et le paramètre lié au système respiratoire comprend un ou plusieurs éléments parmi une compliance du système respiratoire et une résistance du système respiratoire.

3. Procédé de surveillance de la ventilation d'un patient selon la revendication 2, **caractérisé en ce qu'**il comprend en outre :

le fait d'afficher le degré de contribution de chaque paramètre de ventilation ;

de préférence, le degré de contribution de chaque paramètre de ventilation est affiché d'une ou plusieurs manières parmi un mot, un nombre, un caractère, un tableau, un diagramme ou une icône.

4. Procédé de surveillance de la ventilation d'un patient selon l'une quelconque des revendications 1 à 3, **caractérisé en ce qu'**il comprend en outre :

le fait de guider le processus de ventilation mécanique en fonction du degré de contribution.

5. Procédé de surveillance de la ventilation d'un patient selon la revendication 4, **caractérisé en ce que** le fait de guider le processus de ventilation mécanique en fonction du degré de contribution comprend :

le fait d'indiquer de diminuer le paramètre de ventilation selon un classement du degré de contribution ou de commander de diminuer le paramètre de ventilation selon un classement du degré de contribution, lorsqu'il est déterminé que l'énergie qui agit sur le système respiratoire du patient durant le processus de ventilation mécanique dépasse un premier seuil.

6. Procédé de surveillance de la ventilation d'un patient selon la revendication 4, **caractérisé en ce que** le fait de guider le processus de ventilation mécanique en fonction du degré de contribution comprend :

le fait d'indiquer d'augmenter le paramètre de ventilation selon un classement du degré de contribution ou de commander d'augmenter le paramètre de ventilation selon un classement du degré de contribution, lorsqu'il est déterminé que l'énergie qui agit sur le système respiratoire du patient durant le processus de ventilation mécanique est inférieure à un second seuil.

7. Procédé de surveillance de la ventilation d'un patient selon la revendication 4, **caractérisé en ce que** le fait de guider le processus de ventilation mécanique en fonction du degré de contribution comprend :

le fait d'estimer et de fournir l'énergie qui agit sur le système respiratoire du patient durant le processus de ventilation mécanique qui est réglée conformément à une commande de réglage d'un paramètre de contrôle de ventilation.

8. Procédé de surveillance de la ventilation d'un patient selon la revendication 7, **caractérisé en ce que** le fait de guider le processus de ventilation mécanique en fonction du degré de contribution comprend en outre :

le fait de déterminer s'il faut envoyer une alarme en fonction de l'énergie estimée qui agit sur le système respiratoire du patient durant le processus de ventilation mécanique.

9. Procédé de surveillance de la ventilation d'un patient selon la revendication 4, **caractérisé en ce que** le fait de guider le processus de ventilation mécanique en fonction du degré de contribution comprend :

le fait de déterminer s'il faut envoyer une alarme en fonction du degré de contribution de chaque paramètre lié au système respiratoire.

10. Appareil de surveillance de la ventilation d'un patient, comprenant :

un capteur de pression, lequel est configuré pour acquérir une pression d'un patient durant un processus de ventilation mécanique ;

un capteur de débit, lequel est configuré pour acquérir un débit gazeux du patient durant le processus de ventilation mécanique ; et

un processeur, lequel est configuré pour acquérir la pression du patient durant le processus de ventilation mécanique et le débit gazeux du patient durant le processus de ventilation mécanique, et pour calculer, en fonction de la pression acquise et du débit gazeux acquis, une énergie qui agit sur le système respiratoire du patient durant le processus de ventilation mécanique ;

où le processeur est en outre configuré pour acquérir un paramètre de ventilation ;

**caractérisé en ce que** le processeur est en outre configuré pour déterminer un degré de contribution du paramètre de ventilation à l'énergie qui agit sur le système respiratoire du patient durant le processus de ventilation mécanique ;

où la pression reflète une pression qui agit sur différents sites d'un système respiratoire du patient durant le processus de ventilation mécanique.

11. Appareil de surveillance de la ventilation d'un patient selon la revendication 10, **caractérisé en ce que** le paramètre de ventilation comprend un paramètre de contrôle de ventilation et/ou un paramètre lié au système respiratoire ; où le paramètre de contrôle de ventilation comprend un ou plusieurs éléments parmi un volume courant, un débit gazeux, une pression d'entraînement, une pression positive en fin de respiration, une fréquence respiratoire et un ratio respiratoire, et le paramètre lié au système respiratoire comprend un ou plusieurs éléments parmi une compliance du système respiratoire et une résistance du système respiratoire.

12. Appareil de surveillance de la ventilation d'un patient selon la revendication 10 ou la revendication 11, **caractérisé en ce que** le processeur est en outre configuré pour guider le processus de ventilation mécanique en fonction du degré de contribution.

13. Appareil de surveillance de la ventilation d'un patient selon la revendication 12, **caractérisé en ce que**, afin de guider le processus de ventilation mécanique en fonction du degré de contribution, le processeur est en outre configuré pour :

indiquer de diminuer de préférence le paramètre de ventilation ayant le plus fort degré de contribution parmi les paramètres de contrôle de ventilation, ou commander de diminuer de préférence le paramètre de ventilation ayant le plus fort degré de contribution parmi les paramètres de contrôle de ventilation, lorsqu'il est déterminé que l'énergie qui agit sur le système respiratoire du patient durant le processus de ventilation mécanique dépasse un premier seuil ; et/ou

indiquer d'augmenter de préférence le paramètre de ventilation ayant le plus faible degré de contribution parmi les paramètres de contrôle de ventilation, ou commander d'augmenter de préférence le paramètre de ventilation ayant le plus faible degré de contribution parmi les paramètres de contrôle de ventilation, lorsqu'il est déterminé que l'énergie qui agit sur le système respiratoire du patient durant le processus de ventilation mécanique est inférieure à un second seuil.

14. Appareil de surveillance de la ventilation d'un patient selon la revendication 12, **caractérisé en ce que**, afin de guider le processus de ventilation mécanique en fonction du degré de contribution, le processeur est en outre configuré pour :

estimer et fournir l'énergie qui agit sur le système respiratoire du patient durant le processus de ventilation mécanique qui est réglée conformément à une commande de réglage d'un paramètre de contrôle de ventilation, et déterminer s'il faut envoyer une alarme en fonction de l'énergie estimée qui agit sur le système respiratoire du patient durant le processus de ventilation mécanique ; ou

afin de guider le processus de ventilation mécanique en fonction du degré de contribution, le processeur est en outre configuré pour :

déterminer s'il faut envoyer une alarme en fonction du degré de contribution de chaque paramètre lié au système respiratoire.

15. Appareil de surveillance de la ventilation d'un patient selon la revendication 10, **caractérisé en ce que** l'appareil de surveillance de la ventilation d'un patient est un moniteur de patient, un module de surveillance de patient ou un appareil de ventilation médical ; et/ou

l'appareil de surveillance de la ventilation d'un patient comprend en outre un affichage, lequel est configuré pour afficher le degré de contribution de chaque paramètre de ventilation.

FIG.1

FIG.2

Expiratory
branch 213a

213c

214a

Patient

211

Inspiratory
branch 213b

214b

212

Gas

| Memory 215 | Processor 50 | Display 70 |

FIG.3

Expiratory
branch 340a

340c

Patient

311

Inspiratory
branch 340b

Anesthetic drug output apparatus

330

Respiration assistance apparatus

320

312

Gas

| Memory 350 | Processor 50 | Display 70 |

FIG.4

FIG.5

FIG.6

Energy(J/min)

4.70

84%

0.88

16%

Power$_C$　　　　Power$_R$

FIG.7 (a)

Power$_R$

0.88
16%

4.70
84%

Power$_C$

FIG.7 (b)

| Acquiring a gas flow rate of a patient during a mechanical ventilation process |
|---|

100

| Acquiring a pressure of the patient during the mechanical ventilation process |
|---|

200

| Calculating, according to the acquired pressure and the acquired gas flow rate, energy which acts on the respiratory system of the patient during the mechanical ventilation process |
|---|

300

| Acquiring a ventilation parameter |
|---|

400

| Determining a degree of contribution of the ventilation parameter to the energy which acts on the respiratory system of the patient during the mechanical ventilation process |
|---|

500

FIG.8

Acquiring a gas flow rate of a patient during a
mechanical ventilation process

100

Acquiring a pressure of the patient during the
mechanical ventilation process

200

Correcting the acquired pressure of the patient

210

Calculating, according to the acquired pressure and the
acquired gas flow rate, energy which acts on the
respiratory system of the patient during the mechanical
ventilation process

300

Acquiring a ventilation parameter

400

Determining a degree of contribution of the ventilation
parameter to the energy which acts on the respiratory
system of the patient during the mechanical ventilation
process

500

FIG.9

Acquiring a gas flow rate of a patient during a mechanical ventilation process

~100

Acquiring a pressure of the patient during the mechanical ventilation process

~200

Calculating, according to the acquired pressure and the acquired gas flow rate, energy which acts on the respiratory system of the patient during the mechanical ventilation process

~300

Acquiring a ventilation parameter

~400

Determining a degree of contribution of the ventilation parameter to the energy which acts on the respiratory system of the patient during the mechanical ventilation process

~500

Displaying the degree of contribution of each ventilation parameter

~600

FIG.10

Acquiring a pressure of a patient during a mechanical ventilation process

~100

Acquiring a gas flow rate of the patient during the mechanical ventilation process

~200

Correcting the acquired pressure of the patient

~210

Calculating, according to the acquired pressure and the acquired gas flow rate, energy which acts on the respiratory system of the patient during the mechanical ventilation process

~300

Acquiring a ventilation parameter

~400

Determining a degree of contribution of the ventilation parameter to the energy which acts on the respiratory system of the patient during the mechanical ventilation process

~500

FIG.11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007028921 A1
- US 2009062674 A